# EUROPEAN PATENT APPLICATION

(11) **EP 2 399 581 A1**
(43) Date of publication of application: **28.12.2011**
(21) Application number: 11170065.4
(22) Date of filing: 15.02.2008
(51) Int. Cl.: A61K 31/485, A61K 31/49, A61K 31/554, A61K 9/20

(54) **Drug detoxification protocol using microdosing**

(30) Priority: 15.02.2007 US 675560
(62) Divisional of application: 08002803.8
(71) Applicant: Slater, Kenneth C., Manchester MA 01944 (US); Richardson, Brenda E., Manchester MA 01944 (US); Connors, Scott M., Methuen MA 01844 (US)
(72) Inventor: Slater, Kenneth C., Manchester MA 01944 (US); Richardson, Brenda E., Manchester MA 01944 (US); Connors, Scott M., Methuen MA 01844 (US)
(74) Representative: Richaud, Fabien

(57) **Abstract**

This invention relates to use of an opioid receptor agonist in microdose quantities to reduce or eliminate the withdrawal symptoms associated with discontinuing drug use. The dosage of the agonist is reduced over an extended period of time.

## Description

### FIELD OF THE TECHNOLOGY

This invention relates to methods to decrease the withdrawal symptoms a patient suffers of discontinuing drug use.

### BACKGROUND

In the past, methods of helping opioid drug dependencies consisted of treatments with methadone and/or Naltrexone. Naltrexone, however, did not help a patient with the severe withdrawal symptoms and methadone on many occasions proved to be as much of an addiction as the original drug the patient abused. More recently, Buprenorphine has been used as a potential treatment for opiate addiction. However, existing formulations and methods have been limited in their success and compliance rate.

### SUMMARY

The invention provides an improvement over existing methods of drug-based treatment of addiction to narcotics such as opiate drugs. Unlike other detoxification protocols, the methods described herein yield a completion rate is greater than 98%, where completion means a narcotic free state. For example, the rate is 97, 95, 92, 90, 8-0. 85%. This treatment approach reduces or eliminates withdrawal symptoms of addiction to a narcotic agent. The method is carried out by identifying a subject with a history of habitual narcotic use; and administering to the subject a plurality of doses of an opioid receptor agonist at intervals of every 6-120 hours over a period of at least four weeks. In the case of opioid receptor agonist, buprenorphine, the interval is every 24-48 hours. In instances where the person is being taken off of a narcotic such as Oxycodone, the time span between doses varies, the interval is typically shorter, e.g., every 6, 12, or 18 hours.

The method is suitable for a variety of addictive conditions characterized by alteration of brain function via the opioid receptor system such as alcohol and opiates or derivatives thereof. The subject is a young adult or older adult individual, a Hidden Addict, or an Iatrogenic Addict. Each of the doses in the series is temporally decreased in a stepwise manner, an each incremental dose reduction does not exceed 1.0 mg. This microdose strategy is administered over an extended period of time. Optionally, individuals with a history of significant narcotic consumption undergo a pretreatment period in which the dose of the agonist is reduced by 2.0 mg or more, e.g., 5.0 mg, 8.0 mg, per dose interval. At doses of the agonist in the mg range, e.g., 20 mg or less (e.g., 16 mg or less), serial dose reductions are in the range of 0.5-0.01 mg per day. For example, the temporal decrease in dose is less than or equal to 0.5, 0.1, 0.05, 0.025, 0.01 mg every other day, i.e., a dose reduction takes place every 48 hours. Such a dose reduction protocol reduces symptoms of withdrawal; in the event that withdrawal symptoms appear, the dose is maintained or the dose backs up to the previous incremental dose.

In preferred embodiments the agonist is buprenorphine or a derivative thereof, and the duration of treatment is at least 2, 3, or 4 months. To monitor progress, individuals undergoing treatment are evaluated for physical symptoms and/or psychological symptoms of withdrawal at least once every 7 days. Such evaluations occur every 3 days, every 2 days, daily, or twice a day. Symptoms of withdrawal include primary symptoms such as vomiting, sweating, salivation as well as secondary symptoms such as craving.

In some cases, the narcotic to which an individual has developed an addiction is a prescription pain relief medication. Pain relief medication is prescribed to an individual who has been injured, undergone surgery, or is suffering from a disease associated with pain such as cancer or HIV/AIDS. The invention includes a method of treatment for discontinuation of pain management. Such a method includes a schedule of narcotic drug medication and/or a schedule of buprenorphine or other opiate administration, and the schedule of narcotic drug medication exceeds a period of 2 weeks. In some cases, the individual has been on narcotic pain medication for 1, 2, 3, 4, 5, 6, 8, 12 months or more. Physical withdrawal symptoms typically occur after an individual has been taking a narcotic pain medication for approximately 1 month; however, psychological withdrawal symptoms may develop after only days or weeks of pain medication. Buprenorphine or the narcotic pain relief medication itself is administered subsequent to the pain relief narcotic drug prescription in a series of doses, which are temporally decreased in a stepwise manner not to exceed an incremental reduction of 0.25, 0.1, 0.05, or 0.01 mg per day. The method of discontinuing pain management is commenced when a physician diagnoses a patient that is experiencing or is at risk of experiencing symptoms of withdrawal upon cessation of the pain medication. In an alternative method, the pain relieve medication itself is reduced according to the microdose strategy described herein. Doses in the first phase are therapeutic for pain relief, whereas doses of drug in the second phase of the program are sub-therapeutic for pain relief. Instead, the therapeutic goal is avoidance of withdrawal symptoms, and the doses and decreasing dose schedule are therapeutic for inhibition of withdrawal symptoms until the drug is ceased entirely.

A plurality of compositions containing the opioid agonist is packaged in a series of decreasing dosage units, each of which differ by less than 10% in size, shape, or color, wherein the amount of each of said dosage units in a series of doses does not exceed 0. 25, 0.1, 0.05, or 0.01 mg. Preferably, the compositions differ by less than 5, 2, or 1% or are identical. Alternatively, the size of the composition, e.g., pill, capsule, lozenge, increases, e.g., by 0.5-5%, as the dose of active ingredient, e.g., buprenorphine decreases. To deter misuses, the compositions are preferably perishable, i.e., the product dissolves, becomes unpalatable, or otherwise unsuitable for consumption after a predetermined duration of time. The compositions are preferably formulated as a sublingual lozenge or troche, but are also formulated as hard candy, a capsule, a tablet, liquid, microspheres, or a transdermal patch. The composition is also administered intramuscularly or intravenously, e.g., using an i.v. pump.

Also within the invention is a method of reducing withdrawal symptoms of addiction to a narcotic agent by identifying a subject with a history of habitual narcotic use and administering to the subject a dose of a composition comprising a µ opioid agonists, buprenorphine, or a derivative thereof at intervals of every 24-120 hours over a period of at least four weeks. The dose of buprenorphine is temporally decreased in a stepwise manner, and the dose reduction does not exceed 30% of the previous dose.

In each of the methods described herein, the composition is a µ opioid agonist, e.g., a partial agonist or a full agonist. The partial agonist includes a compound with the structure of Formula I. Preferably, the partial agonist is buprenorphine. The temporal decrease in dose of buprenorphine includes a dose reduction by about 10% or less (e.g., 7, 5, 2, 1. 0.5%) every other day at doses of buprenorphine over about 1.00 mg per day. The temporal decrease in dose of buprenorphine includes a dose reduction between about 1-30 % every other day at doses of buprenorphine less than 1.00 mg per day. For example, the temporal decrease in dose of buprenorphine comprises a dose reduction by less than or equal to an amount selected from the group consisting of 30%, 25%, 20%, 15%, 10% 5%, and 1% of the previous dose. At below 2 mg of drug, the incremental dose reduction is generally reduced by increments in ranges of less than 10% going as low as 1% or less on occasion when the actual doses are less than 1 mg. This dose reduction method is applicable for opioid receptors agonist drugs such as Buprenorphine as well as narcotics such as Oxycontin, Fentanyl, and other narcotics listed in Table 1 below.

A method of treating members of a population for an addiction to a narcotic substance is carried out by administering to an individual a protocol that includes a schedule of buprenorphine doses with each of the doses of buprenorphine being temporally decreased in a stepwise manner not to exceed 0.5 mg every other day. Given that monitoring of physical and psychological symptoms of withdrawal is carried out frequently, the population is defined by a 150 mile radius of a treatment center.

Alternatively, a method of treating members of a population for an addiction to a narcotic substance is carried out by administering to an individual a protocol comprising a schedule of buprenorphine doses remote from a treatment center utilizing an electronic, video, or computer communication system. The doses of buprenorphine are temporally decreased in a stepwise manner not to exceed 0.5 mg every other day, and the population is evaluated via a real-time video conference with a treatment provider such as a physician, and wherein medication is dispensed at a location remote from the physician by a machine or dispensing device.

In another example, the method of detoxification is carried out by administering to an individual who is suffering from addiction to alcohol or a narcotic drug a protocol prescribed by a treating physician comprising a schedule of administrations of a composition comprising a µ-opioid receptor agonist. The protocol is coded on a treatment card and the composition is dispensed by a machine or dispensing device adapted to receive and process the treatment card. The treatment card is an encoded card such as a credit card. The machine communicates with the treatment physician or a supervising physician. To facilitate monitoring of symptoms, the treatment physician is video-linked to a location of the dispensing machine, e.g., real time video conferencing. The machine identifies the individual by retinal scan, fingerprint scan, password, or treatment card swipe. The machine maintains a graphic record or log of a transaction or a series of transactions using the treatment card. In some examples, the medication or composition administered contains an identification tag such as a radio-frequency identification tag or serial number. The use of the treatment card is suspended if the composition is not consumed by the individual at the site of administration or if medication abuse is detected, e.g., as evidenced by outside reports from family and significant others, self reports, or "positive" drug tests. Optionally, the machine detects a blood alcohol level, pulse, or blood pressure of the individual.

Also within the invention is a method for administering prescribed medication, which includes the steps of: a) receiving at a data center, an initial input by a patient from a treatment facility such as a kiosk, wherein the initial input is a identification and a password; b) selecting an overall treatment plan designated to coordinate with the initial input; c) sending the overall treatment plan to the client terminal; d) requiring patient inputs based on communication of the overall treatment plan from the data center, wherein the patient inputs are vital monitoring, status questionnaire, treatment announcements, treatment updates, treatment instructions, and payment; e) sending the patient inputs to the data center; f) processing the patient inputs at the data center; g) sending medication administration instructions to the treatment kiosk; h) administering medication to the patient at the treatment kiosk. Patient's input information received at the data center is further sent to an individual therapist and a home treatment center of the patient or a law enforcement agency. A primary treatment center, e.g., a group treatment center, sends information to the data center for the patient.

Other features and advantages of the invention will be apparent from the following description of the preferred embodiments thereof, and from the claims. All references cited herein are hereby incorporated by reference.

### BRIEF DESCRIPTION OF THE DRAWINGS

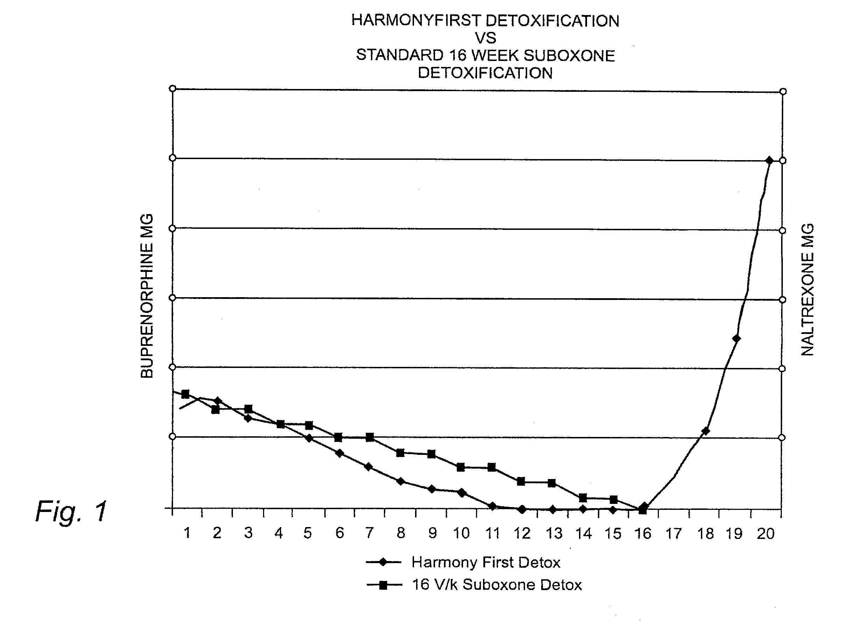

Figure 1 is line graph showing a comparison of a standard detoxification program and a microdose protocol.

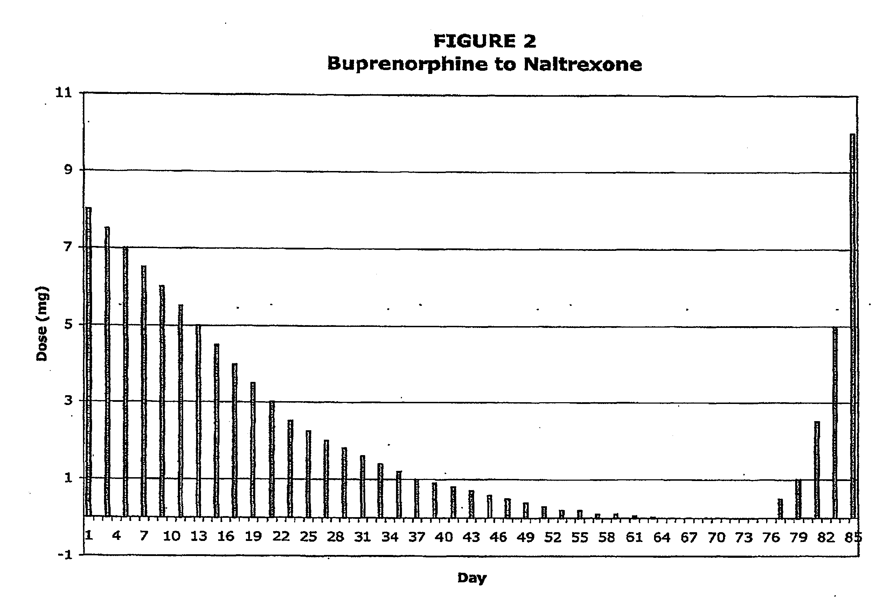

Figure 2 is a bar graph showing a shallow slope incremental decrease of buprenorphine dose. The dose was reduced by .25 to .5 mg every other day in doses over 2.5 mg. After this dose progression, the dose was decreased by .25 mg to 2 mg and then .2 mg every other day to 1 mg and then .1 mg every other day to .1 mg. then .05, .025, .01 then "blanks for 4-6 days then Naltrexone .1 mg.
Figure 3 is a line graph showing the rate of decline of buprenorphine administered to a patient over the course of treatment.
Figures 4 A-C are scales for assessing withdrawal symptoms. Figure 4A is the Subjective Opiate Withdrawal Scale (SOWS). Figure 4B is the Objective Opioid Withdrawal Scale (OOWS). Figure 4C is the Short Opiate Withdrawal Scale (ShOWS), where the scoring is represented by None = 0, Mild = 1, Moderate = 2, and Severe = 3.
Figure 5 is a diagram showing communications associated with the Smart Treatment Machine (STM) and Treatment Kiosk System. The diagram includes decisions made at various points during the process of patient interaction with the STM.

### DETAILED DESCRIPTION

Dependence on psychoactive substances or opioid drugs has been a major health issue across the globe for many years. Among users, there is an increase in the transmission of HIV and Hepatitis C, which, in affect, increases health care costs for the user but also for the community they effect.

A person can become dependent on psychoactive substances with regular drug use in two manners, physically and psychologically. Physically, the brain becomes used to or adapts to the presence of the drug in order to function "normally". Psychoactive drugs affect the central nervous system and alter what the brain considers normal function. At the beginning of drug use, the user experiences a euphoria because the drug affects the nucleus accumbens, or the brain's "pleasure center". Neurons in the nucleus accumbens use the same neurotransmitter as dopamine, although each drug affects this area differently, drugs either stimulate dopamine release or enhance its activity, directly or indirectly. Initially the body only needs a minimal amount of a substance to function normally and attain the state of euphoria, however, over time and with chronic use, the body builds a tolerance for the drug and therefore needs more of the drug to function normally. Additionally, chronic drug use results in less and less stimulation of the nucleus accumbens until eventually it produces no state of euphoria. This physical dependence is a combination of an increased tolerance to a drug and a physical need for the drug to function.

Physical dependence is different from addiction, in that addiction describes the psychological and behavioral aspects of drug use. In addition to the altered brain function, addiction also affects a drug user's perception, mood, consciousness, and behavior. While a person uses drugs, their perception of the environment around them is different than a person who does not use drugs. Drug use becomes a necessity and, therefore, affects the person's daily activities, habits and the people in which they surround themselves.

Long term drug use leads to changes in the nucleus accumbens as well as other regions of the brain. Symptoms of craving and tolerance are mediated by levels of ΔFosB and transcription factor cAMP response element-binding protein (CREB). Aberrant levels of these proteins are present in addicted individuals and normalize following abstinence from the substance to which the individual is addicted. The methods described herein lead to gradual normalization of these parameters over an extended period of time. The brain adapts to chronic narcotic use and then it readapts to sobriety. However, the brain "remembers' physiologically a drug dependent state and reverts to the "addicted" state if it is reexposed to opiates.

Drug rehabilitation is a term for the process of medical and/or psychotherapeutic treatment, for dependency on psychoactive substances, e.g., alcohol, prescription drugs, cocaine, heroin, or amphetamines. The general goal of the drug rehabilitation is drug cessation and avoidance of the psychological, legal, social, and physical consequences associated with reducing and, discontinuance drug use.

There are various types of drug rehabilitation programs with different methods of helping a person to end their drug use. Examples of these programs are residential treatment (in-patient), out-patient, local support groups, extended care centers, and sober houses. One example of this type of drug rehabilitation is the twelve-step program where a member has to admit they do have a problem and work from there, using the support of other members in the group to recover from their drug-use. This program, while helpful for some drug users, has been ineffective for others. These types of programs generally do not medicinally assist in the actual reduction of withdrawal.

### Symptoms of withdrawal

Withdrawal refers to the signs and symptoms that appear when a drug user is physically dependent on a drug and drug use is suddenly discontinued or decreased in dosage. The appearance of withdrawal symptoms can be within a few hours or after several days of discontinuation. The time depends on the drug's elimination half-life. The discontinuation of drug use produces a state of dysphoria, because the nucleus accumbens activity declines below normal levels. These subnormal levels are characterized by depression, anxiety, and craving. Other common signs of withdrawal are increased heart rate and/or blood pressure, tremors, nausea, hallucinations, body aches, and excessive sweating, diarrhea, night terrors, restless leg syndrome, vomiting, dehydration, formications (crawling skin), as well as vivid or disturbing dreams of drug use. Some of the withdrawal symptoms themselves require hospitalization, such as hallucinations and seizures. Additionally, a combination of the symptoms may drive a person to suicide.

Even though a person may no longer be physically dependent, they still may be addicted psychologically to drug use, or the compulsion to use drugs. Psychological withdrawal varies from person to person, but generally, the longer and more persistent the drug use the more intense these psychological symptoms can be.

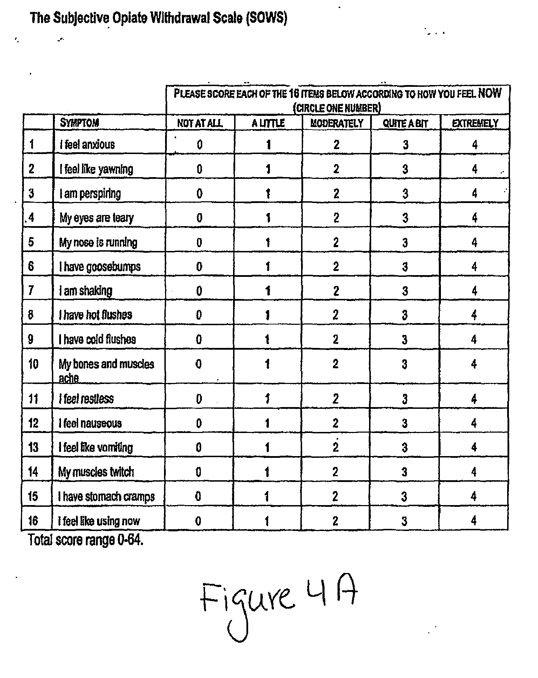

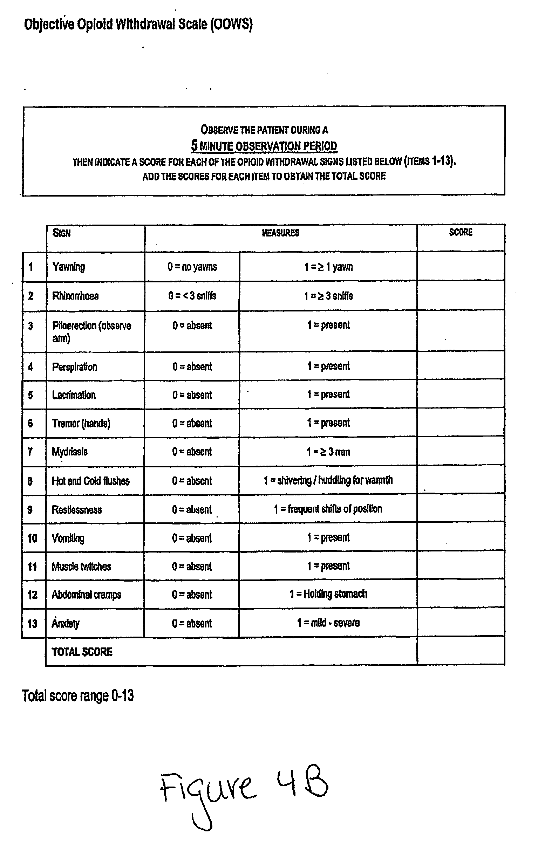

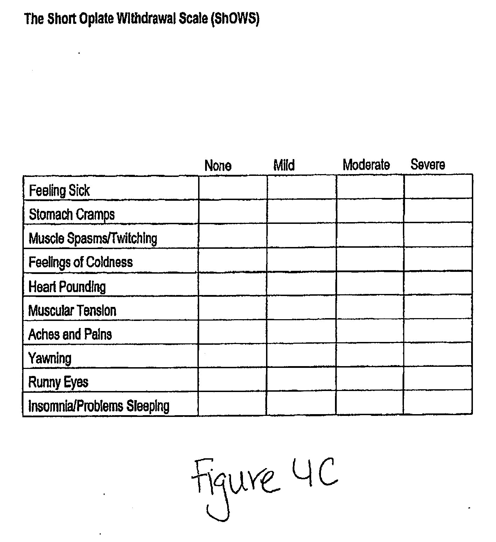

Scales for assessing opioid withdrawal are well known in the art, e.g., Handelsman et al., 1987, Am. J. Drug Alcohol Abuse13(3):293-308; Gossop, M. 1990, Addict Behav. 15(5):487-490. For example, the subjective and objective Opiate Withdrawal Scales assess the severity of withdrawal at the time the scale is administered. These scales are administered at the treatment provider's discretion, e.g., multiple times a day (at 2, 4, 8, 12, 18 hour time points throughout the day), or every 24 hours, 48 hours, 4 days, weekly. The Short Opiate Withdrawal Scale is often abbreviated in literature to SOWA, but referred to herd as ShOWS to avoid confusion with the Subjective Opiate Withdrawal Scale. This scale is typically administered on a daily basis or multiple times a day and assesses the patient's experience of withdrawal in the preceding 24 hours. It includes assessment of sleep disturbance which is an aspect of the withdrawal that is significant to patients. The clinical Opiate Withdrawal Scale (COWS) is the newest of the scales and combines objective and subjective items. Like the SOWS and OOWS, it can be administered multiple times in a day. Exemplary questionnaires are provided in Figs. 4A-C. Although developed for assessment of opiate withdrawal symptoms, these scales are useful to evaluate withdrawal symptoms from other addictive agents as well.

The combination of the withdrawal symptoms, both physical and psychological, increase the probability that a person who is seeking to discontinue use to revert to drug use.

### Characterization of Patient Populations Seeking Treatment

People seeking treatment include a number of different types of patients from different social communities who need treatment for opioid dependence. These characterizations include, but are not limited to the stereotypical drug addict, middle class addicts, young adults between the age of 16-32, and Iatrogenic Addicts. The "drug addict" or Heroin addict is a potentially violent, down and out individual who lies, and steals in order to support his habit. These are the denizens of the inner city hospital emergency rooms late at night who arrive in "pain" or in withdrawal seeking opiate painkillers. These types of addicts are well known to the local community and they have been unsuccessful in current treatment programs, such as Methadone Maintenance.

In contrast, in the middle class population, an addict is generally invisible to their peers, and only the most discerning eye sees that they are suffering from an addiction. For the most part, the drugs of choice in this population are prescription pain killers, and these drugs generally are not taken intravenously. Most are HIV and Hepatitis C negative. The middle class addict is unwilling to "go public" by attending a Methadone program and is willing to get their drugs privately from an addiction physician who prescribes Subutex/Suboxone or from the street. They want to discontinue using but are afraid of withdrawal and the debilitating discomfort of withdrawal and want a private, comfortable detoxification to avoid the withdrawal symptoms and the social ramifications.

The middle class addict is willing to commit some time and funds to this effort, however, they do not want to be studied in a research protocol as that would mean that they would have to go public with their addiction. Fears of damage to social standing and employment prevent enrollment in a public process. As a result, this population of addicts is under reported and under served, and is growing rapidly. The population has grown with the growth of the pain treatment movement and the acceptance of large dose narcotic prescriptions for the treatment of pain. Many in pain treatment programs divert part of their prescriptions to their affluent friends as a way to offset the loss of income caused by their disability.

The middle class patient population can be divided into three large groups. The first is the Youth/Young Adult group. This group is comprised of younger drug dependent individuals age 16 - 32. They are distinguished by their lack of financial resources and their ambivalence around the decision to enter a drug rehabilitation program. They are usually single, or in a relationship where their significant other is a co-user or has been accepting of the drug use. They are employed and hard working, but struggle financially because most of their money is spent buying drugs from the street. For the most part they do not have criminal records and they have not destroyed their relationships with family. Most have very young children, which is the core motivation behind seeking help. The group are educated drug users, in as much as they are familiar with the drugs that are readily available on the street and they are also well informed about legalized drug use programs such as Methadone and Subutex/Suboxone. Most in this group have experimented with a variety of different illicit substances before experiencing a minor injury and receiving a prescription for an opioid painkiller. After several prescriptions the addict becomes dependent on the pain killer and then is cut off from further prescriptions by their doctor. After their prescription ends, they experience withdrawal symptoms and in an effort to avoid the illness they begin buying pain killers off the street. By the time they reach a treatment facility they have been using opiate type drugs for well over 2 years.

Another population group can be called the Hidden Addict. This is a group of people who are seen as successful and competent. They have been able to hide their narcotic use from most of the outside world. Their wealth and hard work has allowed them to support their habit while maintaining a higher standard of living. They are respected in their professions and/or business. Their children are good students and their marriages intact and do not fit the profile of the addict who comes from poverty and an abusive and broken family. Instead, the Hidden Addict is well educated and come from solid homes. Their use starts generally following an injury where they found that they function better if they take a narcotic painkiller. As a result of their education and success, the Hidden Addict has generally supported their habit for years through prescriptions and moved to low risk avenues of use, e.g., buying drugs from the Internet. These, Hidden Addicts, also known as Successful Addicts, generally use very large amounts of drugs, while being socially and vocationally stable. When the Hidden Addict makes the decision to stop drug use they are then committed to success.

Another group of patients treated is seen as Iatrogenic Addicts. This type of addict is mostly middle aged people who, because of medical experience such as cancer treatment, have been placed on a narcotic and have then become addicted to them. They have no prior history of drug use but possibly have a past history of drinking to excess and usually do not have legal problems because of their use. The Iatrogenic Addicts are angry at the medical establishment for putting them into the position where they have lost control of themselves even though they recognize that they were initially treated correctly for pain. They see themselves as being coerced into addiction because their prescribing doctor understands only how to get people to take narcotics but has no understanding of how to help people get off them without experiencing a life altering withdrawal. For the most part the Iatrogenic Addict purchase drugs from others who have been given prescriptions for pain and who wish to augment their incomes by dealing some of the drugs to friends. They are distrustful of current drug treatment programs because they see them as drug use programs. They want to discontinue use and to feel that they are being dealt with honestly and respectfully. Their age and social standing demand this.

In addition the Hidden Addict and the Iatrogenic Addict, other individuals are characterized as substance abusing addicts who are neither Hidden nor Iatrogenic. These are generally people who have a drug dependence, but see themselves as having options and a future. All persons seeking the drug rehabilitation protocol are subject to outside stress. Completion of detoxification is made more challenging by psychosocial stress, medical illness, and underlying psychiatric conditions. These conditions are not always present or visible when treatment is initiated. Evidence that this type of problem is beginning to interfere with detoxification comes when the patient begins to complain of poor sleep and reschedule appointments or arrives late. Early resistance to Naltrexone is also a symptom that a person's commitment to sobriety is wavering. When and if these signs are present they are dealt within the context of the overall treatment and the biweekly visits with the patient.

### Pharmacological intervention for reduction of withdrawal symptoms

Pharmacotherapy has been used in the treatment of drug dependence, because certain medications can significantly reduce physical withdrawal symptoms. Without the withdrawal symptoms, the transition from chronic drug use to a state of being drug free is less intense on the person recovering, making it less likely for the person to revert to drug use. This approach makes it less likely that the patient will drop out of the treatment, and then return to use. The methods increase the likelihood that the patient will complete the process of detoxification and withdrawal, thus increasing his/her chances of not returning to use. Opiates are used as maintenance medications to stave off withdrawal. It is the rapid cessation of daily dosing which causes symptoms. Naltrexone is not used as a maintenance medication. Naltrexone is a blocker rather than a replacement.

The medications have proved especially useful in people who are dependent on addictive drugs, e.g., heroin, oxycodone, hydrocodone, morphine, oxymorphone, fentanyl, and methadone/buprenorphine and other naturally-occurring or synthetic compounds (e.g., alkyloids) that partially or completely activate one or more opioid receptors including Mu, Kappa, Sigma, and Delta receptors.

Medications are used as a maintenance therapy, because they assist in physiologically stabilizing the metabolism of an individual, which has been altered by prolonged drug use as that individual reduces drug use and ceases to use drugs. The maintenance therapy helps to prevent the effects of withdrawal symptoms, which occur when there are no longer any drugs in a recovering drug user's system. Examples of the medications used in the maintenance therapy are methadone, naltrexone, and buprenorphine.

Such medications affect the opiate receptors, as do the opioid drugs, however the medications counteract the effects on the opiate receptors, e.g., µ, κ, and δ opioid receptors. The three receptors are approximately 70% identical with the differences being located at the N and C termini. The G protein binds to the third intracellular loop of the opioid receptor. A locus for opioid activation is the periaqueductal grey region and descending pathways to the spinal cord. The µ-opioid receptor can exist either presynaptically or postsynaptically depending on the cell type and can mediate acute changes in neuronal excitability via "disinhibition" of presynaptic release of GABA. Opioids do not excite descending fibers directly. However, chronic activation of µ-opioid receptor causes the collapse of dendritic spines via post-synaptic mechanisms. The physiological and pathological roles of these two distinct mechanisms may be involved in opioid addiction and opioid -induced deficits in cognition. The µ receptor additionally has a high affinity for enkephalins and β-endorphin but low affinity for dynorphines. Examples of the opioid alkaloids are morphine and codeine.

As initial drug use stimulates the nucleus accumbens and chronic drug use results in less stimulation of this area of the brain, during opiate withdrawal, an increase in the release of the inhibitory neurotransmitter GABA occurs. The effect is mimicked by the adenylyl cyclase activator, forskolin, and inhibited by protein A kinase inhibitors. Therefore, rebound adenylyl cyclase activity in withdrawal may be the fundamental step in eliciting the withdrawal behavior.

Activation of the µ receptor by an agonist, such as morphine, causes analgesia, sedation, reduced blood pressure, itching, nausea, euphoria, decreased respiration, miosis (constricted pupils) and decreased bowel motility. As the body becomes used to the receptor activation, the side effects of euphoria, sedation, and decreased respiration can disappear. Analgesia, miosis and reduced bowel motility, however, persist. The body builds a tolerance to the receptor activation because the effects are caused by different µ-receptor sub types. Stimulation of µ1-receptors block pain, but stimulation of µ2-receptor causes respiratory depression and constipation. Chronic activation of the µ -receptors is analogous to chronic use of opioid drugs. The body builds a tolerance to opioid drugs due to the effects of repeated activation of the µ-receptors and a chronic drug user needs more drugs to feel the effects, however, some side effects remain because they stimulate the µ2-receptors.

The tolerance for respiratory depression and is the primary way a chronic drug user can overdose, less common is a circulatory collapse. However, opioid overdoses can be reversed with the use of an opioid antagonists, such as naltrexone or naloxone. When these opioid antagonists are used to treat a drug overdose they function as inverse agonists.

Thebaine is an opiate alkaloid. Thebaine and paramorphine are minor constituents of opium and are is chemically similar to both morphine and codeine, but rather than having depressant effects, thebaine has a stimulatory effect. Thebaine is not generally used therapeutically, but it is converted into a variety of compounds, including, oxycodone, oxymorphone, nalbuphine, naloxone, Naltrexone, buprenorphine and etorphine. Changes to the morphinan nucleus can result in low efficacy partial agonists and simplification of morphine structure leads to methadone.

Methadone is a synthetic opioid also used as an analgesic that can be delivered both orally and intravenously. Drawbacks of methadone therapy include development of dependence on the drug. At the proper dosing, methadone can reduce the need for a drug such as heroin, but the person recovering is at risk of becoming dependent on methadone.

Naltrexone is an opioid receptor, which is used in the management of alcohol and opioid dependence. Generally the drug is used in rapid detoxification where the opioid-receptor blockade is induced while the patient is in a state of impair consciousness or under anesthesia. Naltrexone, however, does not affect the craving a person may experience during the recovery process. Protocols that utilize Naltrexone for opioid rehabilitation can be complicated and challenging for an out-patient based sobriety treatment.

### Buprenorphine and Related Compounds

Buprenorphine is also an opioid drug and exhibits partial agonist and antagonist actions toward opioid drugs. If buprenorphine overlaps with consumption of another opioid drug, buprenorphine acts as an antagonist by binding to the receptor, thereby inhibiting binding of an opiate and induced abrupt and severe withdrawal symptoms. Thus although it may precipitate withdrawal symptoms in people dependent on other opioids, it behaves as a µ-opioid receptor agonist alone, which causes a less euphoric effect compared to a full agonist, such as methadone. Additionally, Buprenorphine assists with the reduction and/or prevention of withdrawal symptoms unlike Naltrexone.

Buprenorphine has a unique pharmacological characteristic in that, buprenorphine includes moderate intrinsic activity, high affinity to and slow dissociation from µ-opioid receptors. The antinociceptive effect of buprenorphine is mainly mediated by activation of the µ-opioid receptors. Norbuprenorphine is an N-dealkylated metabolite of buprenorphine and is a likely contributor to the overall pharmacology of buprenorphine. Norbuprenorphine provides antinociceptive efficacy similar to buprenorphine. Another derivative of buprenorphine is HS-599, which is a didehydroderivative of buprenorphine that displays high affinity and good selectivity for µ-opioid receptors. Additionally, HS-599 behaves as a partial agonist able to antagonize morphine antinociception in a dose-dependent manner. BU48 us also a ring-constrained analog of buprenorphine, although BU48 has a high affinity to µ-opioid receptors like buprenorphine, unlike buprenorphine BU48 has a binding affinity to κ and δ opioid receptors as well.

The IUPAC name of buprenorphine, the structure of which is shown below, is (2S)-2-[(-)-(5R,6R,7R,14S)-9a-cyclopropylmethyl-4,5-epoxy-6,14-ethanomorphinan-7-yl]-3-hydroxy-6-methoxy-3,3-dimethylbutan-2-ol. (CAS #N02AE01). INN designation: N-cyclopropylmethyl-7&-[1-(S)-hydroxy-1,2,2-trimethylpropyl]6,14-endoethan o-6,7,8,14-tetrahydronororipavine).

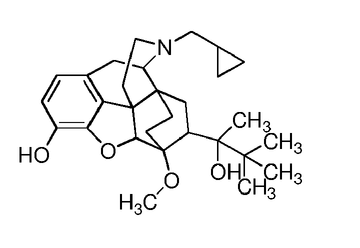

The invention provides a compound having the formula Formula I: 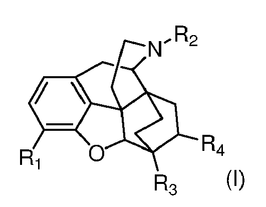 or a pharmaceutically effective salt, solvate, hydrate, or prodrug thereof, wherein R₁ and R₃ are, independently selected from H, hydroxyl, straight chain or branched alkyl, cycloalkyl, straight chain or branched alkenyl, cycloalkenyl, allyl, trifluoromethyl, carboxyalkyl, carboxyamino, aryl, heteroaryl, benzyl, alkoxy, amino, alkylamino, and thioalkyl; R₂ is selected from H, straight chain or branched alkyl, cycloalkyl, straight chain or branched alkenyl, cycloalkenyl, allyl, trifluoromethyl, carboxyalkyl, carboxyamino, aryl, heteroaryl, benzyl; and
R₄ is selected from straight chain or branched alkyl, cycloalkyl, straight chain or branched alkenyl, cycloalkenyl, and straight chain or branched alkynyl, further wherein R₄ is substituted or unsubstituted.

The compound is a salt. The compound is a solvate. The compound is a hydrate. The compound is a prodrug. R₁ is H. R₁ is hydroxyl. R₁ is amino. R₁ is alkylamino. R₁ is alkoxy. R₁ is methoxy. R₁ is straight chain alkyl. R₁ is methyl. R₁ is ethyl.

R₂ is straight chain or branched alkyl. R₂ is methyl. R₂ is ethyl. R₂ is propyl. R₂ is isopropyl. R₂ is methylcycloalkyl. R₂ is methylcyclopropyl. R₂ is methylcyclobutyl.

R₃ is H. R₃ is hydroxyl. R₃ is alkoxy. R₃ is methoxy. R₃ is ethoxy. R₃ is straight chain alkyl. R₃ is methyl. R₃ is ethyl.

R₄ is straight chain alkyl. R₄ is methyl. R₄ is ethyl. R₄ is propyl. R₄ is butyl. R₄ is branched alkyl. R₄ is isopropyl. R₄ is isobutyl.

R₄ is unsubstituted. R₄ is substituted. R₄ is monosubstituted. R₄ is disubstituted. R₄ is trisubstituted. R₄ is substituted with hydroxyl or halogen. R₄ is substituted branched alkyl. R₄ is selected from 2,2-dimethylpropane-1,1-diol, 3,3-dimethylbutan-2-ol, 2,3-dimethylbutan-2-ol, 3-methylbutan-2-ol, and 3-methylbutane-2,3-diol.

Definitions pertaining to chemical entities and moieties are provided below.

"Alkyl" includes saturated aliphatic groups, including straight-chain alkyl groups (*e.g*., methyl, ethyl, propyl, butyl, pentyl, hexyl, heptyl, octyl, nonyl, decyl), branched-chain alkyl groups (*e.g*., isopropyl, tert-butyl, isobutyl), cycloalkyl (*e.g*., alicyclic) groups (*e.g*., cyclopropyl, cyclopentyl, cyclohexyl, cycloheptyl, cyclooctyl), alkyl substituted cycloalkyl groups (e.g., methylcyclopropyl), and cycloalkyl substituted alkyl groups.

"Aryl" includes groups with aromaticity, including 5- and 6-membered "unconjugated", or single-ring, aromatic groups that may include 0, 1, 2, 3, or 4 heteroatoms, as well as "conjugated" or multicyclic systems with at least one aromatic ring. Examples of aryl groups include benzene, phenyl, pyrrole, furan, thiophene, thiazole, isothiazole, imidazole, triazole, tetrazole, pyrazole, oxazole, isooxazole, pyridine, pyrazine, pyridazine, and pyrimidine, and the like. Furthermore, the term "aryl" includes multicyclic aryl groups, *e.g*., tricyclic, bicyclic, *e.g*., naphthalene, benzoxazole, benzodioxazole, benzothiazole, benzoimidazole, benzothiophene, methylenedioxyphenyl, quinoline, isoquinoline, napthridine, indole, benzofuran, purine, benzofuran, deazapurine, or indolizine. Those aryl groups having heteroatoms in the ring structure may also be referred to as "aryl heterocycles", "heterocycles," "heteroaryls" or "heteroaromatics". The aromatic ring can be substituted at one or more ring positions. Aryl groups can also be fused or bridged with alicyclic or heterocyclic rings, which are not aromatic so as to form a multicyclic system (*e.g*., tetralin, methylenedioxyphenyl).

"Alkenyl" includes unsaturated aliphatic groups analogous in length and possible substitution to the alkyls described above, but that contain at least one double bond. For example, the term "alkenyl" includes straight-chain alkenyl groups (*e.g.*, ethenyl, propenyl, butenyl, pentenyl, hexenyl, heptenyl, octenyl, nonenyl, and decenyl), branched-chain alkenyl groups, cycloalkenyl (*e.g.*, alicyclic) groups (*e.g.*, cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. "Alkynyl" includes unsaturated aliphatic groups analogous in length and possible substitution to the alkyls described above, but which contain at least one triple bond. For example, "alkynyl" includes straight-chain alkynyl groups (*e.g.*, ethynyl, propynyl, butynyl, pentynyl, hexynyl, heptynyl, octynyl, nonynyl, decynyl), branched-chain alkynyl groups, and cycloalkyl or cycloalkenyl substituted alkynyl groups.

The term "alkoxy" or "alkoxyl" includes substituted and unsubstituted alkyl, alkenyl, and alkynyl groups covalently linked to an oxygen atom. Examples of alkoxy groups (or alkoxyl radicals) include methoxy, ethoxy, isopropyloxy, propoxy, butoxy, and pentoxy groups.

The terms "heterocyclyl" or "heterocyclic" group include closed ring structures, *e.g*., 3, 4, 5, 6, 7, 8, 9, or 10-, or 4, 5, 6, or 7-membered rings, which include one or more heteroatoms. In one embodiment, the ring structure is a 5 or 6-membered ring. "Heteroatom" includes atoms of any element other than carbon or hydrogen. Examples of heteroatoms include nitrogen, oxygen, sulfur and phosphorus.

Heterocyclyl groups can be saturated or unsaturated and include for example, pyrrolidine, oxolane, thiolane, piperidine, piperazine, morpholine, lactones, lactams such as azetidinones and pyrrolidinones, sultams, and sultones. Heterocyclic groups *e.g.,* pyrrole and furan can have aromatic character. Heterocyclic groups include fused ring structures such as quinoline and isoquinoline. Other examples of heterocyclic groups include pyridine and purine.

The term "hydroxy" or "hydroxyl" includes groups with an -OH or -O-.

The term "halogen" includes fluorine, bromine, chlorine, iodine, etc. The term "perhalogenated" generally refers to a moiety wherein all hydrogens are replaced by halogen atoms.

The structural formula of the compound represents a certain isomer for convenience in some cases, but the invention includes all isomers such as geometrical isomer, optical isomer based on an asymmetrical carbon, stereoisomer, tautomer and the like which occur structurally and an isomer mixture and is not limited to the description of the formula for convenience, and may be any one of isomer or a mixture. Therefore, an asymmetrical carbon atom may be present in the molecule and an optically active compound and a racemic compound may be present in the present compound, but the invention is not limited to them and includes any one. In addition, a crystal polymorphism may be present but is not limiting, but any crystal form may be single or a crystal form mixture, or an anhydride or hydrate. Further, so-called metabolite which is produced by degradation of the present compound *in vivo* is included in the scope of the present invention.

The structure of some of the compounds of the invention includes asymmetric (chiral) carbon atoms. Isomers arising from such asymmetry are included within the scope of the invention, unless indicated otherwise. Such isomers can be obtained in substantially pure form by classical separation techniques and by stereochemically controlled synthesis. The compounds of this invention may exist in stereoisomeric form, therefore can be produced as individual stereoisomers or as mixtures.

"Isomerism" means compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center".

"Chiral isomer" means a compound with at least one chiral center. It has two enantiomeric forms of opposite chirality and may exist either as an individual enantiomer or as a mixture of enantiomers. A mixture containing equal amounts of individual enantiomeric forms of opposite chirality is termed a "racemic mixture". A compound that has more than one chiral center has 2ⁿ⁻¹enantiomeric pairs, where n is the number of chiral centers. Compounds with more than one chiral center may exist as either an individual diastereomer or as a mixture of diastereomers, termed a "diastereomeric mixture". When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al, Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J., Chem. Educ. 1964, 41, 116).

"Geometric Isomers" means the diastereomers that owe their existence to hindered rotation about double bonds. These configurations are differentiated in their names by the prefixes cis and trans, or Z and E, which indicate that the groups are on the same or opposite side of the double bond in the molecule according to the Cahn-Ingold-Prelog rules.

Further, the structures and other compounds discussed in this application include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

The terms "crystal polymorphs" or "polymorphs" or "crystal forms" means crystal structures in which a compound (or salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions. For example, using different solvents or different solvent mixtures for recrystallization; crystallization at different temperatures; various modes of cooling, ranging from very fast to very slow cooling during crystallization, and the like. Polymorphs are also obtained by heating or melting the disclosed compounds followed by gradual or fast cooling. The presence of polymorphs is determined by solid probe nuclear magnetic resonance spectroscopy, infrared spectroscopy, differential scanning calorimetry, powder X-ray diffraction, and other techniques known to one skilled in the art.

Additionally, the compounds of the present invention, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvates" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative", refers to compounds that have a common core structure, and are substituted with various groups as described herein. For example, all of the compounds represented by Formula I are buprenorphine derivatives, and have Formula I as a common core.

The compounds of the invention are capable of forming salts. All of these forms are also contemplated within the scope of the claimed invention.

"Pharmaceutically acceptable salt" of a compound means a salt that is pharmaceutically acceptable and that possesses the desired pharmacological activity of the parent compound.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, *e.g.*, glycine, alanine, phenylalanine, arginine, etc.

Other examples include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The invention also encompasses salts formed when an acidic proton present in the parent compound either is replaced by a metal ion, *e.g.*, an alkali metal ion, an alkaline earth ion, or an aluminum ion; or coordinates with an organic base such as ethanolamine, diethanolamine, triethanolamine, tromethamine, N-methylglucamine, and the like.

It should be understood that all references to pharmaceutically acceptable salts include solvent addition forms (solvates) or crystal forms (polymorphs) as defined herein, of the same salt.

The pharmaceutically acceptable salts of the present invention can be formed from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are used.

The number of atoms protonated and counterions associated with the salt can be controlled and depends on the number of acidic/basic atoms in the parent compound and amount of acid which is used to treat the parent compound. In one embodiment of the invention, the monohydrochloride salt of the parent compound is formed upon treatment with hydrochloric acid. In another embodiment, the dihydrochloride salt of the parent compound is formed upon treatment with hydrochloric acid.

Lists of suitable salts are found in *Remington's Pharmaceutical Sciences,* 18th ed. (Mack Publishing Company, 1990). For example, salts can include, but are not limited to, the hydrochloride and acetate salts of the aliphatic amine-containing, hydroxyl amine-containing, and imine-containing compounds of the present invention. Throughout the specification, the term buprenorphine refers to the base as well as any pharmaceutically acceptable salt, e.g., buprenorphine hydrochloride. Formulations are further described in U.S. Patent No. 4,935,428; 4,661,492; and 6,995,169.

### Opioid Receptor Interactions

The analgesic effect of Buprenorphine is due to partial agonist activity at µ-opioid receptors, i.e., when the molecule binds to a receptor, it is only partially activated in contrast to a full agonist such as morphine. Buprenorphine also has very high binding affinity for the µ receptor such that opioid receptor antagonists (e.g. naloxone) only partially reverse its effects. The drug's mean half-life is approximately 35-37 hours. For this reason, patients switching to buprenorphine are required to abstain from the previous opioid for at least several half-lives of the given substance.

Buprenorphine is also a κ-opioid receptor antagonist, and partial/full agonist at the recombinant human ORL1 nociceptin receptor. Buprenorphine hydrochloride can be administered by a number of different routes, e.g., intramuscular injection, intravenous infusion, transdermal patch, sublingual tablet, tablet, capsule or other soluble menas, (e.g., hard candy. Oral administration is limited due to very high first-pass metabolism. Buprenorphine is metabolized by the liver, via the CYP3A4 isozyme of the cytochrome P450 enzyme system, into norbuprenorphine (by N-dealkylation) and other metabolites. The metabolites are further conjugated with glucuronic acid and eliminated mainly through excretion into the bile. The elimination half-life of buprenorphine is 20-73 hours (mean 37).

The main active metabolite, norbuprenorphine, is a δ-opioid receptor and ORL1 receptor agonist, µ- and κ-opioid receptor partial agonist, but buprenorphine antagonizes its effects

Buprenorphine, when used in a drug rehabilitation protocol, is administered via sublingual troches for the treatment of opioid addiction. The method of medically supervised withdrawal of addicts from narcotics described herein is both safe and effective and achieves a success rate of greater than 90% when taken to completion of the dose reduction schedule. In previous treatment regimens, the duration of withdrawal symptoms while using buprenorphine was three to five days with doses of buprenorphine ranging from 8.0 to 3.0 mg. Such protocols had limited success. Therefore, studies were undertaken to re-evaluate and modify the doses and schedule of administration. The resulting microdose strategy was developed after extensive observation of patient responses to numerous dosing strategies. The microdose methods described herein carried out over an extended period, e.g., months rather than days or weeks, yields a completion rate of approximately 96%.

### The microdose approach to reduce or eliminate withdrawal symptoms Oxycontin

The microdose strategy is also useful to inhibit withdrawal symptoms associated with abrupt cessation of pain relief medication such as Oxycontin (IUPAC name: 4, 5-epoxy-14-hydroxy-3-methoxy-17-methylmorphinan-6-one; chemical formula: C₁₈H₂₁NO₄; CAS Registry Number 76-42-6) or Fentanyl (IUPAC name: N-(1-phenethyl-4-piperidyl)-N-phenyl-propanamide; chemical formula: C₂₂H₂₈N₂O; CAS Registry No. 437-38-7) and other narcotic drugs shown in Table 1 below. Either buprenorphine or the pain relief narcotic itself is administered in a microdose schedule at doses that are sub-therapeutic for pain relief but therapeutic for inhibition of withdrawal symptoms.

**Table 1**

| | |
|---|---|
| Actiq | Methadose |
| Alfenta | Morphine sulfate |
| Avinza | MS Contin |
| Buprenex | Nalbuphine |
| buprenorphine | Nubaine |
| butorphanol | Numorphan |
| butorphanol nasal | Opana |
| codeine phosphate | Opana ER |
| codeine sulfate | Oramorph SR |
| Darvon | oxycodone |
| Darvon-N | OxyContin |
| Demerol | OxyFast |
| DepoDur | OxyIR |
| Dilaudid | Palladone |
| Dolophine | propoxyphene HCl |
| Duragesic | Roxanol |
| ETH-Oxydose | Roxicodone |
| fentanyl transdermal | Stadol |
| Fentora | Stadol NS |
| hydromorphone | Sufenta |
| Kadian | sufentanil |
| Levo-Dromoran | Talwin |
| levorphanol | Talwin NX |
| meperidine | Ultiva |
| methadone | |

### Methods of Administration

A method of treatment includes self-administration of medication; however, this presents the risk of a patient hoarding the medication to distribute to others to ingest more than their prescribed dose. One approach to prevent or reduce hoarding of any medication administered during treatment includes formulations of the tablet, capsule, troche, or other composition for oral administration to render it unpalatable. Planned deterioration of the medication includes a number of methods, including developing a bad taste, the tablet disintegrates, or another method that makes it difficult for a patient to hoard the medication for any reason other than their prescribed intention.

Another way to prevent medication hoarding or to ensure that there are vacant treatment appointments available for a patient is to increase access to drug detoxification and maintenance services would through an ATM type machine that would dispense medication, also called a smart treatment machine (STM). A person can initially come to a treatment center for evaluation and the development of a structured treatment plan, also known as a treatment protocol. The treatment plan includes a plan for both medication as well as counseling. The length of the treatment would be defined based on the patient and the symptoms presented. Patient expectations also would be established at the initially evaluation, including attendance at a given number of counseling sessions per week, maintenance of a job, and drug tests, among other concerns or considerations that are a part of the treatment protocol.

Once at treatment plan has been agreed upon, the subscriber, also known as the patient or client, is issued a card, similar to a credit card, that has embedded in it the details of the treatment plan. The card then allows the subscriber to go to a facility that has a medication dispensing machine and swipe the card and get the correct amount of medication. To ensure security in that the actual patient is receiving the proper medication and using their own card, the card, after it is swiped the subscriber would have to place their thumb on a fingerprint pad or eye to a retinal scanner and if this matches what is embedded on the card the medication would be dispensed. At the same time a photo record would be kept of each transaction. The medication would be dispensed in a form that would be difficult to divert. In the case of with a prescribed medication such as Methadone, it would be dispensed into a cup that has a tracking device built into it. If the cup is not replaced before the patient opens the doors of the treatment facility to leave, the patient's card is suspended and the patient would need to have the card re-validated at the treatment center where they had their initial consultation.

The smart treatment machine would also be useful in maintaining known addicts who are also known to have a pain condition. Currently, it is impractical to dispense daily supply of pain medications as it may more likely lead to over use or diversion. By only allowing an individual to receive a fixed daily dose dispensed within a given time frame daily the risk of abuse and diversion would be decreased. In the case of buprenorphine maintenance the dose could be manufactured in a gelatin like strip where the dosage is calculated by length, which would be cut and dispensed. Unlike a pill, this form of sublingual troche is less likely to be diverted; however, pills can be dispensed as well.

A similar approach is applicable to treatment for alcohol abuse or addiction. After being screened medically, a treatment regimen is developed with a patient in which the patient goes to a medication dispensing machine that optionally includes a breathalyzer along with a blood pressure (BP) and pulse measuring device and a screen. Based on the data programmed into the treatment card, the patient is administered the appropriate medications. The patient participates in interactive videos as well as individual meetings with an alcohol counselor (LADAC). Blood pressure, pulse and blood alcohol levels are monitored and must be within predetermined limits contained on the treatment card. For example, if a blood alcohol level were high, the machine would inform the treating physician and treatment center. If the blood alcohol level was dangerously high, a pre-set number, the medication dispensing machine will call the police or another emergency responder and/or a family member. If blood pressure and/or pulse do not return to normal after a few days then the participant is instructed to contact the treatment physician. If the patient does not make contact, the physician can contact the patient or the patient's family so that the medical crisis is averted.

By using such a smart treatment machine or dispensing device, clinical expertise is expanded beyond both geographic and talent pool boundaries. A patient who has elected to detoxify from alcohol at a treatment center far from their home area has access, as part of their aftercare, ongoing virtual sessions with a counselor from the patients initial treatment center. By doing this with an STM local provider, services are kept up to date and distant providers who might have a deeper understanding of the patient as well as the clinical issue would be able to continue to provide treatment for the patient. Using telephone interview strategies strengthens patient recovery. Treatment of alcohol induced disorder is improved with monitoring as well as follow-up and follow-up support. The STM approach improves monitoring as well as follow-up through notification of local providers if treatment appointments have not been met.

Information obtained or generated by the dispensing machine, in addition to other aspects of the treatment including, the lab, support group attendance, work attendance, among others are sent to the treatment center as well as being incorporated into the patient's or subscriber's digital medical record so that the card and machine reads each patient's record. The dispensing machines are situated in treatment centers and licensed health care facilities as well as pharmacies and other locations such as a medical suite of a cruise ship.

Advantages of such dispensing machines and treatment approaches utilizing such machines is that such a program encourages the drug dependent individual to normalize their life. The patients does not have to think about getting to the treatment facility or the clinic to get their medication, instead the patient has the confidence and knowledge that they can go to any dispensing location to get their prescribed medication, and the treating physician still follows the patients closely. Monitoring includes evaluation of physiological parameters, e.g., pulse, blood pressure, and blood sugars. Any deviation from the prescribed treatment protocol, or what is considered normal, is easily tracked and instantly brought to the attention of the patient's treatment center and treating physicians and adjustments in care or medication are made accordingly.

A key difference between this form of smart treatment machine and a vending machine is that the STM is apart of an integrated treatment plan. It is the data hub of the pharmacological part of a drug treatment program sending information to clinicians, treatment centers, regulatory agencies and pharmacists. The STM is interactive so that it is capable of requesting drugs screens, subscriber visits with a clinician, treating physician, group, or group leader or other changes in the patient's treatment protocol. For example, if a subscriber comes in to the STM to get their medication, they are informed that their support group will or will not meet that evening or day. Furthermore, subscribers may contact their clinician from the machine. Current machines use pre-filled prescriptions and are simply dispensers. The STM is an integral part of the treatment protocol.

### Development of the microdose treatment protocol to reduce physical symptoms of withdrawal

Buprenorphine troches for the treatment (not maintenance) of opiate dependence were used on a patient that was a 21 years old male who was using I.V. 5-8 bags of heroin daily. He was started on 3.0 mg of buprenorphine and remained on this dose for three days. After a two day period without opiates, he was started on the opiate blocker Naltrexone. Commencement of daily Naltrexone was the end point of treatment. In this way it can be assured that a person is indeed drug free as they are narcotic blocked.

After many such withdrawal attempts, it became clear that a single dose in mg of buprenorphine given over a short period of time would not lead to a sustainable sobriety. Buprenorphine taken sublingually has a mean half-life of 35-37 hrs. If the dose is reduced outside the time frame of the half life, the patient is always experiencing a falling dose and reacts with both physical and emotional symptoms of withdrawal. By reducing the dose slowly, i.e., microdoses within the mean half-life, the patients experience a period where there is little or no decline in biologically available narcotic and thus feel more secure as they are not experiencing even minor withdrawal symptoms.

Accordingly, the drug rehabilitation protocol was altered to reflect a growing clinical understanding of opiate dependence and how buprenorphine is helpful in dealing with reduction of withdrawal symptoms. The alteration included a greater variety of doses ranging from 8.0 to 4.0 mg administered in a deceasing amount over time. At first the dose strengths varied from 8.0 mg administered once daily with a daily reduction of 2.0 mg until to 4.0 mg, and then to 2.0 mg. However during the earliest use of buprenorphine, it was understood that to go from administering 3.0 mg or even 2.0 mg to sobriety was too steep a cut-off point (i.e. patient would experience challenging withdrawal symptoms) and that fixed doses do not serve the patients well.

A fractional buprenorphine dose was administered to reduce the severity of withdrawal symptoms. This strategy was in response to ongoing withdrawal symptoms following the "conclusion" of withdrawal or a "final" 3.0 mg dose of buprenorphine. To mitigate the withdrawal symptoms, a dose of 1.5 mg of buprenorphine was given to the patient. This particular treatment was unsuccessful as the ongoing withdrawal symptoms were too difficult for the patient who relapsed as a result.

A 6.0 mg dose was then added to the protocol. This lengthened the withdrawal period from three days to seven. This change was occasioned by the observation that the drop from 8.0 mg to 4.0 mg was all too frequently accompanied by a significant return of withdrawal symptoms and, at times, a return to use.

Since naltrexone is administered following the cessation of narcotic there can be no residual narcotic in a patient's system, otherwise the naltrexone will push the patient into withdrawal. Therefore, the patient must wait about a week before to start Naltrexone, even with extremely low doses, doses that may be considered to be homeopathic but are high enough to cause withdrawal symptoms if the narcotic is not fully washed out. As necessitated by the recognition that Naltrexone, even administered at 12.5 mg, causes a return of withdrawal symptoms for up to five days after the end of buprenorphine treatment. The patient has to wait at least five days, remaining opiate free, before Naltrexone could start.

### Psychological withdrawal

Another change in the protocol dealt with the growing awareness of the psychology of opiate dependence, or the psychological withdrawal symptoms. Initially, all patients were informed daily of the dose that they were to receive in the office and the proposed dose schedule for their complete detoxification. This clearly caused distress with the patients who would become panicky or anxious upon learning that they would receive a smaller dose today than yesterday and that their treatment would likely end in four days time. The patients were exhibiting the psychological withdrawal symptoms associated with reducing the amount of opioid in their body.

Understanding psychological withdrawal symptoms, and seeing how it complicated the withdrawal process, the protocol was changed to: a) make all of the lozenges look and taste the same and b) absence of discussions regarding dose with patients as it lead to unnecessary anxiety. Instead, the discussion is focused on withdrawal symptoms and craving type symptoms. A craving is a dysphoric affective state that occurs in the process of withdrawal from a drug of dependence in the absence of NVD but may include physical symptoms of pain, malaise, insomnia, headache, anergia, anorexia, or vivid and disturbing dreams of drug use.

Further observations indicated that to end the treatment with a buprenorphine troche and then ask the patient to wait five to seven days before starting on Naltrexone would likely lead to relapse. Therefore, Naltrexone was compounded into fractional doses starting at 1.0 mg. By so doing, the time with no medication is shortened and the risk of any withdrawal symptoms is reduced by creating the blocking of opiate residue by Naltrexone.

Despite these alterations in the treatment protocol the results were still not what were expected. The observation was made that a cut-off of 2.0 mg would cause a return of withdrawal symptoms within 24 hrs and thus the likelihood of a return to drug use before Naltrexone could be started. The data indicated that the downward transition or incremental decrease needed to be smoother. As a result, 1.0 mg lozenges were administered. In addition to buprenorphine, diazepam as well as 25.0-100.0 mg Seroquel for sleep were optionally used to combat anxiety for some patients.

### Determination of the rate of incremental decrease in dosage units

After several years of clinical experience in the use of buprenorphine, a 1.0 mg cut-off was found to be too steep and that a single daily dose of buprenorphine would likely lead to too relapses in treatment. Commercially available buprenorphine (Suboxone & Subutex) is available in strengths of 8.0 mg as well as 2.0 mg. However, that these medications would not suffice for a successful program, because the 2.0 mg dose was too large and too steep cut-off. A 2.0 mg dose reduction of buprenorphine caused significant withdrawal symptoms in an experienced or chronic user of narcotic type medications. As a result, reduction in dosage was be tapered below 1.0 mg as well as using two doses of buprenorphine daily for those patients who had very heavy daily narcotic use patterns (i.e. over 240.0 mg of OxyContin daily).

The use of Diazepam was abandoned in the early phase of withdrawal as it was observed to be too dis-inhibiting (in that patients have an increased impulse to use drugs with judgement impaired by the Diazepam) and prevented outpatients from returning to work in a timely manner. Also observed was that the loss of appetite drastically affect the treatment of patients. The loss of appetite presages a progression of withdrawal symptoms and withdrawal symptoms fears and can lead to relapse or premature termination of treatment among patients. Given this observation in the behavior of patients, olanzepine 5.0-20.0 mg (Zyprexa) was substituted for both Seroquel as well as diazepam for sleep and for appetite stabilization.

The studies provided further insights into the challenges (both physical and psychological) that the patients face indicating a further modification of decreasing dose progressions. Stepwise increments were amended to proceed in the sub 1.0 mg dose range. The taper at this time was 1.0 mg, 0.75 mg, 0.5 mg, to 0.25 mg then a four day period of blank troches followed by an upward taper of troches with Naltrexone going from 0.5 mg of Naltrexone to 10.0 mg of Naltrexone. With serial doses of 0.25 mg of buprenorphine many patients continued to report perceptible and, in some cases, intolerable withdrawal symptoms.

As a result, all of the treatments ended when an individual was successfully started on Naltrexone, i.e., the individual was entirely off of narcotics. The protocol no longer stopped when buprenorphine content of the lozenges was discontinued. Instead, the patients returned daily and were given 'blank' lozenges. After a sufficient period to allow for a total washout of residual buprenorphine, the patients were transitioned to Naltrexone 0.5 mg in lozenge form. This concentration then was gradually increased to 10.0 mg. When a patient reached 10.0 mg Naltrexone in lozenge form, they were moved to Naltrexone PO.

As a result of the ongoing objective patient observations and patient's subjective complaints of withdrawal symptoms, a 0.1 mg dose was introduced. Additionally, a dose of 1.5 mg as well as a dose of 0.75 mg was added to the dose progression series. Although withdrawal complaints were limited, they were still present, and generally in the late phases of treatment. The data indicated that in order to reduce the complaints of withdrawal symptoms, the dose increments had to be further reduced in the sub 2.0 mg taper range. A strategy of 0.1 mg decreases in dose was implemented when the patients reached the 4.0 mg dose range (i.e., 2.0 mg twice a day) and 0.01 mg deceases in the below 1.0 mg range. The effect of this change also to extended the withdrawal period to about six to eight weeks from four weeks. The extended treatment time, allowed for concentrating the treatment on the psychological side of the dependency after the physical dependency subsided. This extension of service allowed for greater focus on the emergence of cravings as the patients approached the 2.0 mg barrier. The 2.0 mg was observe to have the psychological component where the illness began to manifest itself in such a manner that sobriety was threatened. Establishment of a personal dialogue was found to be important in establishing an anchor in recovery and in assuaging a sense of abandonment that many patients experience. Psychological evaluation includes a Socratic dialogue that probes the individuals thought processes including dreams and fantasies of drug use.

To further reduce the physical and psychological symptoms of withdrawal, the rate of taper or stepwise decrease in buprenorphine dose was reduced to 1.0 mg every other day and doses were given twice a day. Although successful for the most part in eliminating withdrawal symptoms, there continued to be a group of patients who were large users of longer acting narcotics such as Methadone and OxyContin that found that they continued to have significant withdrawal symptoms. An induction phase of treatment is expanded from 3-5 days to 5-10 days before the withdrawal process is started. In an effort to eliminate the last vestiges of withdrawal symptoms, the taper was altered to reduce the dose to 5.0 mg every other day. Additionally, the number of sub 0.1 mg doses administered was increased resulting in a transition of almost 100% of our patients safely and in a symptomless manner from the opiate buprenorphine to the blocker Naltrexone. The change further lengthened the treatment course from two months to three or more months. The length of treatment was dependent upon the daily dose of narcotics taken prior to entering treatment.

The methods described herein have been successful in individuals who were using massive amounts of pain killer type narcotics, such as doses of Fentanyl as Actiq in excess of 1200 mcgm #10 daily, as well as Methadone and illegal forms of opiates (Heroin 4+ gm IV daily). Patients to be treated include those with the following exemplary drug use profiles: Percocet 500/10mg, 5-30 tabs per day; Vicodin 500/10mg, 5-30 tabs per day; OxyContin 10-80mg, 1-25 tabs per day; Methadone 10-400mg per day; MSContin 100-400mg per day; Fentanyl 75 1200 mcgm per day; Ultram 50-100mg 10-50 tabs per day; Diluadid 4-76mg per day; Demerol 50-1500mg per day; Heroin intranasally or intravenously 1 "bag" to 4 gm per day; and Suboxone 2-32 mg per day.

### Inhibition of Withdrawal Symptoms Associated with Cessation of Non-Opiates

Most medications that have the potential for dependence are dosed so that they will be effective, i.e., they will resolve the target symptom be it pain, anxiety, seizures, depression etc. In contrast, the target symptom of the microdose approach is the withdrawal symptom(s); a therapeutic dose is one that does not cause withdrawal symptoms. Any medication that causes withdrawal symptoms upon abrupt cessation, when withdrawn slowly according to the microdosing schedule confers clinical benefit by reducing or eliminating physiological and/or psychological withdrawal symptoms.

Certain families of medications such as the benzodiazepines (Librium, Valium, Xanax, Klonopin, Ativan, etc) have well known pathways that lead to their withdrawal states. Benzodiazepines that are abruptly stopped cause severe seizures as well as panic attacks. In addition, over time some individuals develop a psychological dependence on this type of medication as well as a physical one. For these individuals, a micro dose approach to cessation is the most clinically sound way to get a person off the drug. This approach especially applicable for those subjects, who have become dependent on this class of medication and who also have a concomitant alcohol dependence since the benzodiazepines and alcohol are seen as similar by the body.

Abrupt cessation of a benzodiazepine in an active drinker can cause that person to increase their alcohol consumption. For example, the effects of Xanax withdrawal (as an example) are described below. What has become clinically apparent with Xanax which appears to be somewhat different than the other benzodiazepines is that the patient's ability to self-detox (longer acting benzodiazepines such as Klonopin/Valium) or be able to be gradually tapered off of the medication is markedly more difficult. Thus once the physiologic dependence has occurred with Xanax, the ability of the patient to discontinue use successfully on their own is quite low, and medical assistance becomes of significant necessity in the majority of cases. The withdrawal syndrome from Xanax and other benzodiazepines are quite similar, with the exception that Xanax has a much higher incidence of panic attack and a bereavement type of emotional lability that is singularly more severe. Since the symptoms are almost all internal, with a few physical or objective manifestations, the diagnosis of it can be very difficult. Patients have a difficult time verbally describing what is occurring, and much of the descriptions often take on a quality or character reminiscent of the emotional or psychiatric problem for which they originally began taking Xanax, rather than a withdrawal symptom.

The withdrawal syndrome is diagnosed based on the following description of symptoms. In the early stage of withdrawal, there is a presentation of a sense of anxiety and apprehension associated with increasing subjective sense of tremor and mild bifrontal headache. This rapidly progresses to feelings of panic-like anxiety with tachycardia and palpitations, as well as a rapidly progressing feeling of de-realization, which is an altered sense of reality. Symptoms may also be associated with marked startle response and a general amplification of most sensory input. As the withdrawal syndrome progresses, there is a marked disturbance of proprioception, with difficulty in ambulation relative to feeling "dizzy" and "unsteady," needing to use reference and physical objects to steady oneself. With the proprioceptive problem increasing in severity simple acts such as swallowing, signing one's name, talking or even buttoning a shirt can become extremely difficult. many patients at this stage describe hot/cold sensations and generalized myalgia. There is also a progession of extreme emotional lability with sudden outbursts of crying or near panic levels of anxiety and fearfulness which will have sudden onset without clear connection to external events. Associated with this are frequent hypochodriacal fears of morbid consequence from the sensations they are feeling, such as fear of heart attack or stroke. Patients experience a type of emotional dysphoria which is very difficult for them to verbalize, but which come very close by cumulative description to a bereavement type of feeling that is very painful emotionally. Additionally, the amplification of almost all sensory information coming into the brain, other than that of taste, can produce many bizarre misinterpretation of sensory stimulation ranging from feeling one's teeth rotating in their sockets to parts of their bodies disassociating or "falling off".

As the withdrawal symptoms progress, illusionary and hallucinatory phenomena, predominately of a visual nature, begin to manifest themselves, initially with patterns and geometric shapes, and then into full-formed complex visual hallucinations. These symptoms also often become associated with delusions of bodily dysfunction or discorporation. It is frequent and common for the patient to conclude that he is having a nervous breakdown, or "going crazy" as an attempt to try to understand the process at hand, not understanding it as withdrawal phenomena. With further progression, disorientation to person and place occurs with full delirium, and eventually withdrawal will finalize with tonic-clonic major motor seizure activity, generally singular in nature, although several cases of status have been reported. The last triad of symptoms, hallucinosis, delirium and seizure, are classified as major symptoms of Xanax withdrawal and other symptoms of drugs in this category. The withdrawal syndrome can take from months, e.g., 2-6 months, up to two years to fully resolve.

Another class of medication that is favorably withdrawn using the microdose approach is SSRI antidepressants, e.g., Paxil and Zoloft, as well as the non SSRI antidepressant Wellbutrin. The methods are useful to safely and effectively reduce and then cease dependence on antidepressants such as those that inhibit neuronal uptake of serotonin, norepinephrine, and/or dopamine. The listed withdrawal symptoms for Paxil are noted in the below chart and are representative of other SSRI type antidepressants. Complaints of insomnia and "body shock" are terrifying to a person wishing to get off of this medication but finding that they subject to highly unpleasant and disruptive consequences of sudden cessation such as:. intense insomnia; extraordinarily vivid dreams; extreme confusion during waking hours; intense fear of losing sanity; steady feeling of existing outside of reality (depersonalization at times); memory and concentration problems; panic attacks; severe mood swings (heightened irritability / anger); suicidal thoughts; dizziness/vertigo; feeling of shocks (similar to mild electric one, running the length of your body); unsteady gait; slurred speech; headaches; profuse sweating, e.g.., at night; muscle cramps; blurred vision; breaking out in tears; hypersensitivity to motion, sounds, smells; decreased appetite; nausea; abdominal cramping, diarrhea; loss of appetite; and/ or chills/ hot flashes.

Effexor is another antidepressant with a withdrawal syndrome that is reduced or eliminated using a microdose strategy. Prior to the invention, individuals seeking to cease medication struggled for years, e.g., two years. The schedule involved months skipping one day in between dose,. nine months later two days in between, and as of three months ago, 3 days in between, followed by 4 days in between., Withdrawal symptoms included a sensation of buzzing (electrical impulses) upon eye or head movement, nausea, difficulty concentrating, forgetfulness, numbness in feet, twitching in the leg, disruptive sleep, night sweats, blurred vision, hallucinations, and difficulty with daily activities including work and driving. Microdosing as described herein obviates the need to take off a week of work and allows the individual being treated to function without marked distress. Often such symptoms are rarely or not reported to physicians, and physicians currently have no strategy for dealing with the problem.. Microdosing gives the clinician the ability to rapidly and safely get their patients off of medications that pose a risk of substantial discomfort if they are not discontinued at a slow, steady and gradual pace. The strategy is also applicable to other antidepressants, e.g., the monoamine oxidase (MAO) inhibitor class of drugs such as Nardil, Parnate, and Marplan., which may be associated with seizures if withdrawn rapidly.

Anti-seizure medication also poses a withdrawal risk, e.g., seizures, if abruptly terminated. However, microdose reduction schedules for such drugs, e.g., barbiturates such as phenobarbitols, Dilantin, Depacote, reduce or eliminate the risk of adverse effects of cessation of the drug.

### Personalized Precision Treatment Kiosk/Module

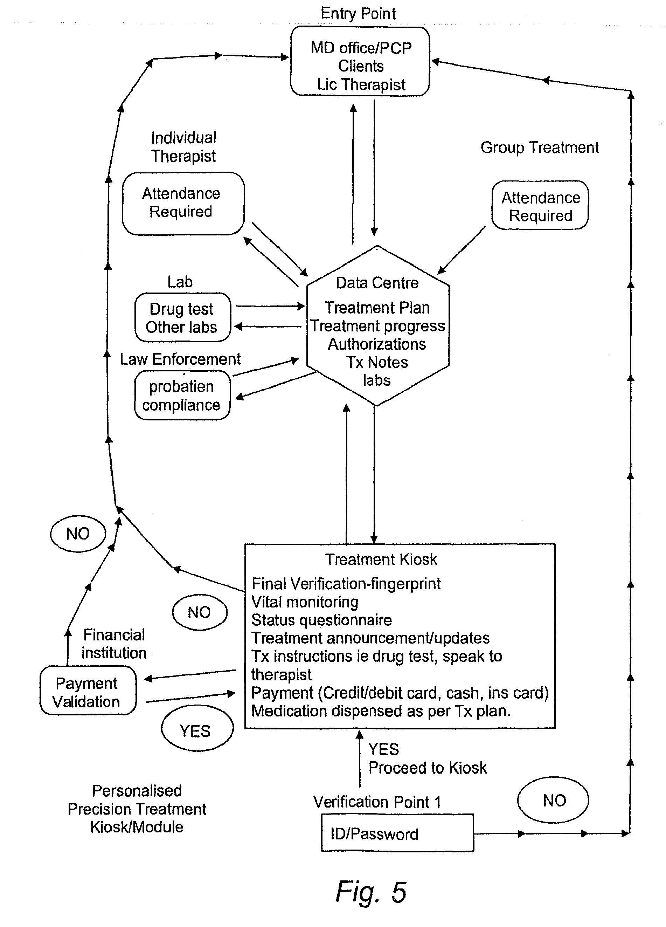

The Personalized Precision Treatment Kiosk/Module (TPPTKM) brings together the functionality of current and future data management technology and modem automated medication dispensing technology (Fig. 5). Utilizing relational database technology, Internet based information system technology, long distance monitoring technology, card scanning, and information bedding technology, telemedicine based technology, banking technology, multi language translation technology, as well as security screening and monitoring technology, with the clinical protocols and knowledge management information technology as well as best practices algorithms used to assess, assign, treat, and monitor those patients who are required to be on monitored treatment protocols. This system serves to promote the improved management of patients such as those on Methadone Maintenance and other maintenance programs requiring strict supervision. The system allows better monitoring of individual patient progress and improves the functioning of the office or clinic responsible for care by improving efficiency, and quality. The ability to have payment at point of service allows better financial controls for both patient and clinic. A significant advantage of the system is expansion of access to treatment for patients and improvement of the quality of the service at the Methadone/Maintenance clinics. The monitoring aspect encourages private medical offices and other venues to provide a drug detoxification service without adding a new and unmanageable burden to their office schedules. The system permits microdosing to abstinence for individuals on Methadone and other drugs. Another benefit is the reduction of diversion. The current practice in Methadone Clinics allows for once only daily dosing which historically has resulted in a great amount of "over use" thus increasing illicit drug use and "failure". Since this system does not require staffing, a twice daily protocol is possible, thereby reducing failure. The data developed by this system is used for the early recognition of problems in individual patients and clinics in a way that has not been seen before, enhanced communication of clinical data, and enhanced quality improvement. Such an integrated data management and treatment module system is useful to individuals investigating new treatments by providing a mechanism for collection and management of objective data from several sources. Often these different sources have information valuable to a good result, but which are rendered useless because there is no opportunity to integrate this information with other data generated by other bodies.

At the ENTRY POINT - (EP), the participant is assessed and deemed appropriate for use of the system. The Physician/clinic completes a uniform treatment and assessment plan which is transmitted to the Data Center (CPU). This information resides on the secure central server. Data generated from outside components of the treatment plan also resides in the CPU. Such data includes lab results, attendance at treatment programs, parole visits etc. These data points are entered through standard protocols from the points of origin, i.e., labs, treatment programs and monitoring agencies as well as other specified treatment programs (group therapy, Individual Counseling, Intensive Out Patient Programs etc). This evaluation system includes evaluation protocols, which select those patients most likely to benefit from having access to this technology thus creating the likelihood of better outcomes.

Having been evaluated, the office/clinic enters the participant data into the system. A Precision Treatment Participant (TPTC) card would be generated. The card is embedded with a PIN and password. This card allows the participant to pass through Verification Point #1. Once inside the participant inserts the card again, this time into the machine. Whilst at Verification Point #2, a mechanism for the scanning of identification characteristics unique to the participant allows the participant to interact with the machine and ultimately receive the medication. These identifying characteristics include, but are not be limited to fingerprints, retinal scans, facial recognition, PIN numbers, and demographic data etc. Optionally, the patient is photographed, and the image entered into the system. As with standard ATMs, every transaction is photographed and recorded. Also at this point (V#2), payment is taken utilizing ATM technology (credit card/debit card,) cash processing technology, or swipe card technology (e.g., transit cards in public transportation systems).

Vital Sign Monitoring for each individual via electronic blood pressure machine and pulse oxymetry is carried out to ensure the participant is appropriate for treatment. Values that fall outside the set parameters for that individual would result in the medicine being held and or the individual referred back to the office/clinic for further testing and closer evaluation. While the Vital Signs are determined, the participant is directed to complete a Status Questionnaire. This assessment tool evaluates the participant's status over the last twenty four hours with respect to Activities of Daily Life (ADL's), sleep hygiene, appetite, Mental Status, and withdrawal symptoms (Figs. 4A-C). As noted, an acuity score is generated based on the scoring of the participant's responses. This score determines the system response based on the treatment protocol for that patient.

Upon entering the TTPTKM, the participant once again goes through a validation process including ID and password as well as unique physical identifier. Once verified, they receive a clinical update message such as "see your therapist or MD" or a reminder to attend group that evening or go to the lab for a urine. Then, a clinical questionnaire is given and processed. If the psychological and physical monitoring data (blood pressure (BP), pulse, breathalyzer, "dirty" urines etc.) is satisfactory, the participant is asked for payment, and if payment is accepted, medication is administered.

The Kiosk includes a number of monitoring devices including, but not limited to pupil size indicator, breathalyzer, blood pressure, pulse, and temperature monitor. Additionally, the participant answers questions on a touch screen (Status questionnaire). Results from these evaluations are transmitted to the CPU, where they are rearranged and collated to generate either a Yes (proceed to dispense message) or a No (do not dispense, advise to see clinician message). Intoxication is a reason for not dispensing as are other signs of acute change. Although a participant may have had the drug dispensed, answers to the questions posed may prompt the participant to see the clinician. Further treatment (Tx) instructions are generated at this point in the interaction. At this point the patient receives a receipt.

The CPU sends patient-specific reports back to the treating agency/physician/therapist. Based on this data, alterations in treatment are optionally made. Patients are then contacted and asked to arrange for a reevaluation visit. During these sessions, changes in the treatment plan can be made. For example, a patient (Pt)t that has a positive urine test is requested to see his/her therapist more frequently. Based on such data, the frequency of random urine screens is optionally increased. A patient that reports that she is pregnant on the daily questionnaire would be given special attention and her OB/GYN MD would be asked to participate during this phase of the Patients treatment. Likewise patients who report back through their daily questionnaire that they are experiencing life difficulties and or clinical difficulties would receive appointments to speak with their treatment advisor who would be able to modify the treatment plan. Another instance where the TTPTKM might positively influence practice and disease outcome is when a participant has HIV or Hepatitis C. Missed MD appointments and/or treatment appointments are noted. and the participant is informed that they must get validation from their MD before they will be authorized to get further medication through the TTPTKM. This monitoring system provides better oversight and management of these challenging medical conditions. This system also applies to those individuals testing positive for alcohol while in the kiosk since the concomitant use of alcohol and methadone is contraindicated. Another advantage of the system is a significant reduction of overdose related deaths for those in maintenance programs.

Those inside the TTPTKM who do not pass validation or payment protocols are referred back to the Entry Point where appropriate adjustments can be made. For example, paper money that is rejected is exchanged for newer bills that the scanner would accept. Those who have no payment could revise their treatment plan to go through a mutually acceptable "financial detoxification" program. Data on such treatment ending events are made available to regulatory agencies so that gaps in safety net services can be addressed. Password failures are invited to enter the system legitimately if there is an error in processing their unique identifier information. The CPU can be asked to display a picture of the participant for verification by the person manning the Entry Point. Those who are trying to enter the system without proper authorization are invited to commence evaluation and treatment or be invited to leave the premises. If they agree to evaluation and are deemed acceptable for the KIOSK program, then fingerprints or another unique identifier is scanned and validated. Those that do not pass evaluation may be entered into another route of treatment.

### Example 1

This patient began treatment with being administered a dosage of 6.0 mg of buprenorphine daily, via four lozenges. From there, the dosage of buprenorphine decreased daily by 0.5 mg, until the dosage was 2.0 mg. From there the patient's dosage was decreased 0.2 mg a day until the dosage was 1.0 mg. At that point, the dosage was reduced by 0.1 mg a day and the dosage of 0.2 mg was given for two days. When the patient reached a dosage of 0.1 mg, the dosage was then dropped to 0.05 mg, however, due to the patient suffering from withdrawal symptoms, the dosage was increased to 0.1 mg, which was administered for three days. At which point the dosage was again decreased to 0.05 mg a day for two days. The dosage was then decreased to 0.03 mg, then to 0.01 mg of the last day of treatment with buprenorphine.

The patient was then given a 1.0 mg dosage of Quinine. On the last day the patient was given a 100 mg dosage of Seroquel. A summary of the dosage can be seen in Table 2 below:

**Table 2**

| **Prescription No.** | **Medication** | **Dosage (mg)** | **No. of Lozenges** | **Directions/Frequency** |
|---|---|---|---|---|
| 8412 | Buprenorphine Troche | 6.00 | 4 | Take 1 Troche sublingually Twice daily |
| 8413 | Buprenorphine Troche | 5.50 | 4 | Take 1 Troche sublingually Twice daily |
| 8414 | Buprenorphine Troche | 5.00 | 4 | Take 1 Troche sublingually Twice daily |
| 8415 | Buprenorphine Troche | 4.50 | 4 | Take 1 Troche sublingually Twice daily |
| 8416 | Buprenorphine Troche | 4.00 | 4 | Take 1 Troche sublingually Twice daily |
| 8417 | Buprenorphine Troche | 3.50 | 4 | Take 1 Troche sublingually Twice daily |
| 8418 | Buprenorphine Troche | 3.00 | 4 | Take 1 Troche sublingually Twice daily |
| 8419 | Buprenorphine Troche | 2.00 | 4 | Take 1 Troche sublingually Twice daily |
| 8420 | Buprenorphine Troche | 1.80 | 4 | Take 1 Troche sublingually Twice daily |
| 8421 | Buprenorphine Troche | 1.60 | 4 | Take 1 Troche sublingually Twice daily |
| 8422 | Buprenorphine Troche | 1.40 | 4 | Take 1 Troche sublingually Twice daily |
| 8423 | Buprenorphine Troche | 1.20 | 4 | Take 1 Troche sublingually Twice daily |
| 8424 | Buprenorphine Troche | 1.00 | 4 | Take 1 Troche sublingually Twice daily |
| 8425 | Buprenorphine Troche | 0.90 | 4 | Take 1 Troche sublingually Twice daily |
| 8426 | Buprenorphine Troche | 0.80 | 4 | Take 1 Troche sublingually Twice daily |
| 8427 | Buprenorphine Troche | 0.70 | 4 | Take 1 Troche sublingually Twice daily |
| 8428 | Buprenorphine Troche | 0.60 | 4 | Take 1 Troche sublingually Twice daily |
| 8429 | Buprenorphine Troche | 0.50 | 4 | Take 1 Troche sublingually Twice daily |
| 8430 | Buprenorphine Troche | 0.40 | 4 | Take 1 Troche sublingually Twice daily |
| 8431 | Buprenorphine Troche | 0.30 | 4 | Take 1 Troche sublingually Twice daily |
| 8432 | Buprenorphine Troche | 0.20 | 4 | Take 1 Troche sublingually Twice daily |
| 8433 | Buprenorphine Troche | 0.20 | 4 | Take 1 Troche sublingually Twice daily |
| 8434 | Buprenorphine Troche | 0.10 | 4 | Take 1 Troche sublingually Twice daily |
| 8435 | Buprenorphine Troche | 0.05 | 4 | Take 1 Troche sublingually Twice daily |
| 8729 | Buprenorphine Troche | 0.10 | 4 | Take 1 Troche sublingually Twice daily |
| 8730 | Buprenorphine Troche | 0.10 | 8 | Take 1 Troche sublingually Twice daily |
| 8731 | Buprenorphine Troche | 0.10 | 8 | Take 1 Troche sublingually Twice daily |
| 8732 | Buprenorphine Troche | 0.05 | 8 | Take 1 Troche sublingually Twice daily |
| 8854 | Buprenorphine Troche | 0.05 | 8 | Take 1 Troche sublingually Twice daily |
| 8855 | Buprenorphine Troche | 0.03 | 8 | Take 1 Troche sublingually Twice daily |
| 8856 | Buprenorphine Troche | 0.01 | 8 | Take 1 Troche sublingually Twice daily |
| 8438 | Naltrexone Troche | 0.50 | 3 | Take 1 Troche sublingually Twice daily |
| 8436 | Quinine Troche | 1.00 | 4 | Take 1 Troche sublingually Twice daily |
| 8437 | Quinine Troche | 1.00 | 4 | Take 1 Troche sublingually Twice daily |
| tel | Seroquel | 100.00 | 120 | Tab ii-iv po qHS prn onsomnia |

### Example 2

This patient began treatment with being administered a dosage of 6.0 mg of buprenorphine daily, via nine lozenges. From there the dosage of buprenorphine decreased daily by 0.25 mg, until the dosage was 2.0 mg. From there, the patient's dosage was decreased 0.2 mg a day until the dosage was 1.0 mg. At that point, the dosage was reduced by 0.1 mg a day. When the patient reached a dosage of 0.1 mg, the dosage was then dropped to 0.05 mg, the dosage was then 0.03 mg, and finally 0.01 mg on the final day of treatment with of buprenorphine.

The patient was then given a 1.0 mg dosage of Quinine for two days. On the last day the patient was given a 0.5 mg dosage of Naltrexone. A summary of the dosage can be seen in Table 3 below:

**Table 3**

| **Prescription No.** | **Medication** | **Dosage (mg)** | **No. of Lozenges** | **Directions/Frequency** |
|---|---|---|---|---|
| 9013 | Buprenorphine Troche | 6.00 | 9 | Take 1 Troche sublingually Four Times Daily |
| 9014 | Buprenorphine Troche | 5.75 | 9 | Take 1 Troche sublingually Four Times Daily |
| 9015 | Buprenorphine Troche | 5.50 | 9 | Take 1 Troche sublingually Four Times Daily |
| 9016 | Buprenorphine Troche | 5.25 | 9 | Take 1 Troche sublingually Four Times Daily |
| 9017 | Buprenorphine Troche | 5.00 | 9 | Take 1 Troche sublingually Four Times Daily |
| 9018 | Buprenorphine Troche | 4.75 | 9 | Take 1 Troche sublingually Four Times Daily |
| 9019 | Buprenorphine Troche | 4.50 | 9 | Take 1 Troche sublingually Four Times Daily |
| 9020 | Buprenorphine Troche | 4.25 | 9 | Take 1 Troche sublingually Four Times Daily |
| 9021 | Buprenorphine Troche | 4.00 | 9 | Take 1 Troche sublingually Four Times Daily |
| 9022 | Buprenorphine Troche | 3.75 | 9 | Take 1 Troche sublingually Four Times Daily |
| 9023 | Buprenorphine Troche | 3.50 | 9 | Take 1 Troche sublingually Four Times Daily |
| 9024 | Buprenorphine Troche | 3.25 | 9 | Take 1 Troche sublingually Four Times Daily |
| 9025 | Buprenorphine Troche | 3.00 | 9 | Take 1 Troche sublingually Four Times Daily |
| 9026 | Buprenorphine Troche | 2.75 | 9 | Take 1 Troche sublingually Four Times Daily |
| 9027 | Buprenorphine Troche | 2.50 | 9 | Take 1 Troche sublingually Four Times Daily |
| 9028 | Buprenorphine Troche | 2.25 | 9 | Take 1 Troche sublingually Four Times Daily |
| 9029 | Buprenorphine Troche | 2.00 | 9 | Take 1 Troche sublingually Four Times Daily |
| 9053 | Buprenorphine Troche | 1.80 | 9 | Take 1 Troche sublingually Four Times Daily |
| 9054 | Buprenorphine Troche | 1.60 | 9 | Take 1 Troche sublingually Four Times Daily |
| 9055 | Buprenorphine Troche | 1.40 | 9 | Take 1 Troche sublingually Four Times Daily |
| 9056 | Buprenorphine Troche | 1.20 | 9 | Take 1 Troche sublingually Four Times Daily |
| 9057 | Buprenorphine Troche | 1.00 | 9 | Take 1 Troche sublingually Four Times Daily |
| 9058 | Buprenorphine Troche | 0.90 | 9 | Take 1 Troche sublingually Four Times Daily |
| 9059 | Buprenorphine Troche | 0.80 | 9 | Take 1 Troche sublingually Four Times Daily |
| 9060 | Buprenorphine Troche | 0.70 | 9 | Take 1 Troche sublingually Four Times Daily |
| 9061 | Buprenorphine Troche | 0.60 | 9 | Take 1 Troche sublingually Four Times Daily |
| 9062 | Buprenorphine Troche | 0.50 | 9 | Take 1 Troche sublingually Four Times Daily |
| 9063 | Buprenorphine Troche | 0.40 | 9 | Take 1 Troche sublingually Four Times Daily |
| 9064 | Buprenorphine Troche | 0.30 | 9 | Take 1 Troche sublingually Four Times Daily |
| 9065 | Buprenorphine Troche | 0.20 | 9 | Take 1 Troche sublingually Four Times Daily |
| 9066 | Buprenorphine Troche | 0.10 | 9 | Take 1 Troche sublingually Four Times Daily |
| 9067 | Buprenorphine Troche | 0.05 | 9 | Take 1 Troche sublingually Four Times Daily |
| 9068 | Buprenorphine Troche | 0.03 | 9 | Take 1 Troche sublingually Four Times Daily |
| 9069 | Buprenorphine Troche | 0.01 | 9 | Take 1 Troche sublingually Four Times Daily |
| 9070 | Quinine Troche | 1.00 | 9 | Take 1 Troche sublingually Four Times Daily |
| 9071 | Quinine Troche | 1.00 | 9 | Take 1 Troche sublingually Four Times Daily |
| 9072 | Naltrexone Troche | 0.50 | 4 | Take 1 Troche sublingually Once Daily |

### Example 3

This patient began treatment with being administered a dosage of 7.5 mg of buprenorphine daily, via four lozenges. From there the dosage of buprenorphine decreased daily by 0.5 mg, until the dosage was 2.0 mg. From there, the patient's dosage was decreased 0.2 mg a day until the dosage was 1.0 mg. At that point, the dosage was reduced by 0.1 mg a day. When the patient reached a dosage of 0.1 mg, the dosage was then dropped by 0.02 mg each day until finally the dosage was 0.01 mg on the final day of treatment with of buprenorphine.

The patient was then given a 1.0 mg dosage of Quinine for four days. On the last day the patient was given a 0.5 mg dosage of Naltrexone. A summary of the dosage can be seen in Table 4 below:

**Table 4**

| **Prescription No.** | **Medication** | **Dosage (mg)** | **No. of Lozenges** | **Directions/Frequency** |
|---|---|---|---|---|
| 8892 | Buprenorphine Troche | 7.50 | 4 | Take 1 Troche sublingually Twice Daily |
| 8893 | Buprenorphine Troche | 7.00 | 4 | Take 1 Troche sublingually Twice Daily |
| 8894 | Buprenorphine Troche | 6.50 | 4 | Take 1 Troche sublingually Twice Daily |
| 8895 | Buprenorphine Troche | 6.00 | 4 | Take 1 Troche sublingually Twice Daily |
| 8896 | Buprenorphine Troche | 5.50 | 4 | Take 1 Troche sublingually Twice Daily |
| 8897 | Buprenorphine Troche | 5.00 | 4 | Take 1 Troche sublingually Twice Daily |
| 8898 | Buprenorphine Troche | 4.50 | 4 | Take 1 Troche sublingually Twice Daily |
| 8899 | Buprenorphine Troche | 4.00 | 4 | Take 1 Troche sublingually Twice Daily |
| 8900 | Buprenorphine Troche | 3.50 | 4 | Take 1 Troche sublingually Twice Daily |
| 8901 | Buprenorphine Troche | 3.00 | 4 | Take 1 Troche sublingually Twice Daily |
| 8902 | Buprenorphine Troche | 2.50 | 4 | Take 1 Troche sublingually Twice Daily |
| 8903 | Buprenorphine Troche | 2.00 | 4 | Take 1 Troche sublingually Twice Daily |
| 8904 | Buprenorphine Troche | 1.80 | 4 | Take 1 Troche sublingually Twice Daily |
| 8905 | Buprenorphine Troche | 1.60 | 4 | Take 1 Troche sublingually Twice Daily |
| 8906 | Buprenorphine Troche | 1.40 | 4 | Take 1 Troche sublingually Twice Daily |
| 8907 | Buprenorphine Troche | 1.20 | 4 | Take 1 Troche sublingually Twice Daily |
| 8908 | Buprenorphine Troche | 1.00 | 4 | Take 1 Troche sublingually Twice Daily |
| 8909 | Buprenorphine Troche | 0.90 | 4 | Take 1 Troche sublingually Twice Daily |
| 8910 | Buprenorphine Troche | 0.80 | 4 | Take 1 Troche sublingually Twice Daily |
| 8911 | Buprenorphine Troche | 0.70 | 4 | Take 1 Troche sublingually Twice Daily |
| 8912 | Buprenorphine Troche | 0.60 | 4 | Take 1 Troche sublingually Twice Daily |
| 8913 | Buprenorphine Troche | 0.50 | 4 | Take 1 Troche sublingually Twice Daily |
| 8914 | Buprenorphine Troche | 0.40 | 4 | Take 1 Troche sublingually Twice Daily |
| 8915 | Buprenorphine Troche | 0.30 | 4 | Take 1 Troche sublingually Twice Daily |
| 8916 | Buprenorphine Troche | 0.20 | 4 | Take 1 Troche sublingually Twice Daily |
| 8917 | Buprenorphine Troche | 0.10 | 4 | Take 1 Troche sublingually Twice Daily |
| 8918 | Buprenorphine Troche | 0.08 | 4 | Take 1 Troche sublingually Twice Daily |
| 8919 | Buprenorphine Troche | 0.06 | 4 | Take 1 Troche sublingually Twice Daily |
| 8920 | Buprenorphine Troche | 0.05 | 4 | Take 1 Troche sublingually Twice Daily |
| 8921 | Buprenorphine Troche | 0.03 | 4 | Take 1 Troche sublingually Twice Daily |
| 8922 | Buprenorphine Troche | 0.01 | 4 | Take 1 Troche sublingually Twice Daily |
| 8923 | Quinine Troche | 1.00 | 4 | Take 1 Troche sublingually Twice Daily |
| 8924 | Quinine Troche | 1.00 | 4 | Take 1 Troche sublingually Twice Daily |
| 8925 | Quinine Troche | 1.00 | 4 | Take 1 Troche sublingually Twice Daily |
| 8926 | Quinine Troche | 1.00 | 4 | Take 1 Troche sublingually Twice Daily |
| 8927 | Naltrexone Troche | 0.50 | 4 | Take 1 Troche sublingually Once Daily |

### Example 4

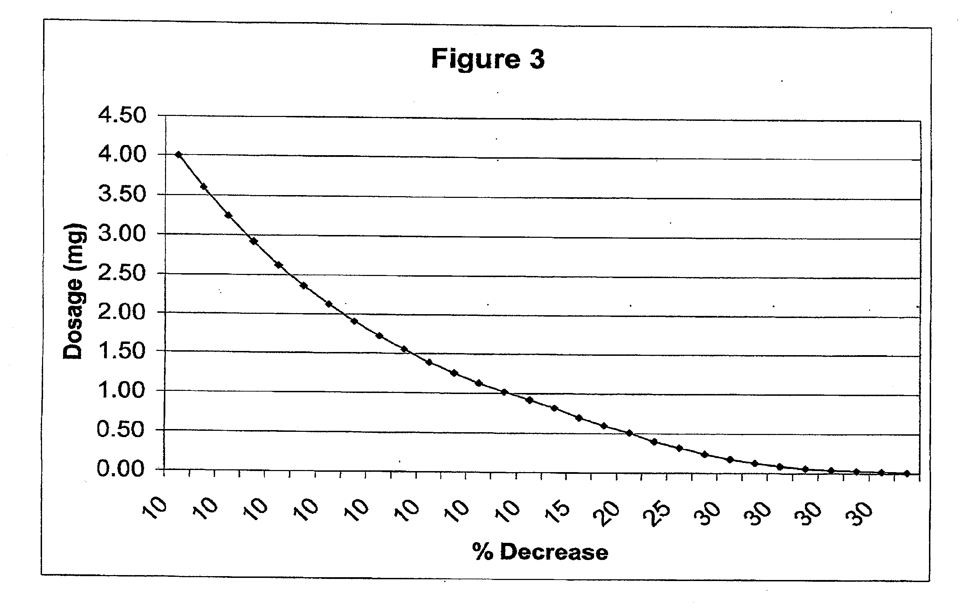

This patient began treatment having buprenorphine being administered a dosage of 4.0 mg of buprenorphine. Typically, a patient is given two doses of medication, at the same amount of buprenorphine, daily spaced approximately 12 hours apart and each doss is given for about two days. From there the each new dosage of buprenorphine decreased overtime by 10%, until dosage 16, at which point, the patient's dosage was decreased 15% for three dosages. At that point, the dosage was reduced by 20% for each dosage over the course of two dosages. The next two dosages were reduced 25%. The dosages from dosage 23 to dosage 30 were reduced by another 30% per dosage. Figure 3 shows a graphical representation of the rate of decline of the amount of buprenorphine administered and a summary of the dosage can be seen in Table 5 below:

**Table 5**

| Dosage No. | Buprenorphine (mg) | % Decrease |
|---|---|---|
| 1 | 4.00 | 10 |
| 2 | 3.60 | 10 |
| 3 | 3.24 | 10 |
| 4 | 2.92 | 10 |
| 5 | 2.62 | 10 |
| 6 | 2.36 | 10 |
| 7 | 2.13 | 10 |
| 8 | 1.91 | 10 |
| 9 | 1.72 | 10 |
| 10 | 1.55 | 10 |
| 11 | 1.39 | 10 |
| 12 | 1.26 | 10 |
| 13 | 1.13 | 10 |
| 14 | 1.02 | 10 |
| 15 | 0.92 | 10 |
| 16 | 0.82 | 15 |
| 17 | 0.70 | 15 |
| 18 | 0.60 | 15 |
| 19 | 0.51 | 20 |
| 20 | 0.40 | 20 |
| 21 | 0.32 | 25 |
| 22 | 0.24 | 25 |
| 23 | 0.18 | 30 |
| 24 | 0.13 | 30 |
| 25 | 0.09 | 30 |
| 26 | 0.06 | 30 |
| 27 | 0.04 | 30 |
| 28 | 0.03 | 30 |
| 29 | 0.02 | 30 |
| 30 | 0.01 | 30 |

Although certain aspects, examples and embodiments have been described above, it will be recognized by the person of ordinary skill in the art, given the benefit of this disclosure, that additions, substitutions, modifications, and alterations of the disclosed illustrative features, aspects, examples and embodiments are possible.

## Claims

1. Opioid receptor agonist for use in a detoxification treatment of drug addiction, said detoxification treatment comprising identifying a subject with a history of habitual narcotic use; and
administering to said subject a plurality of daily dosages of an opioid receptor agonist over a period of at least four weeks, wherein each of said dosages of said opioid receptor agonist is temporally decreased in a stepwise manner, wherein an incremental reduction in dosage does not exceed 1.0 mg, wherein at dosage of the agonist in the mg range, the temporal decrease in daily dosage is in the range of 0.5-0.01 mg per day, and wherein:
- when reaching a daily dosage of 2 mg, said dosage is reduced by 0.2 mg a day until reaching a daily dosage of 1 mg, and
- at that point, the daily dosage is reduced by 0.1 mg a day until reaching 0.1 mg a day.

2. Opioid receptor agonist according to claim 1, wherein at a daily dosage of 0.1 mg, the dosage is reduced by about 0.02 mg each day until reaching the dosage of 0.01 mg a day.

3. Opioid receptor agonist according to claim 1, wherein at a daily dosage of 0.1 mg, the dosage is reduced by 0.05 mg a day to 0.05 mg and then by 0.02 mg a day until reaching the dosage of 0.01 mg a day.

4. Opioid receptor agonist according to claim 2 or 3, wherein, after reaching the dosage of 0.01 mg a day, 1.0 mg dosage of Quinine is given for at least two days, and preferably for four days.

5. Opioid receptor agonist according to claim 2, wherein, after reaching the dosage of 0.01 mg a day, 1.0 mg dosage of Quinine is given for four days.

6. Opioid receptor agonist according to claim 3, wherein, after reaching the dosage of 0.01 mg a day, 1.0 mg dosage of Quinine is given for two days.

7. Opioid receptor agonist according to any of claims 4 to 6, wherein 0.5 mg of Naltrexone is given for one day after the step of administering Quinine.

8. Opioid receptor agonist according to any of claims 1-7, wherein the opioid receptor agonist is administered to a subject over a period of at least thirty days.

9. Opioid receptor agonist according to claim 2, claim 5 or claim 7 when depending on claim 5, wherein the opioid receptor agonist is administered to a subject over a period of thirty-one days.

10. Opioid receptor agonist according to claim 3, claim 6 or claim 7 when depending on claim 6, wherein the opioid receptor agonist is administered to the subject over a period of thirty-four days.

11. Opioid receptor agonist according to any of claims 1 to 10, wherein each of the daily dosages of the opioid receptor agonist is administered to the subject more than once a day, and preferably twice a day or four times a day.

12. Opioid receptor agonist according to claim 2, claim 5, claim 7 when depending on claim 5, or claim 9, wherein each of the daily dosages of the opioid receptor agonist is administered to the subject twice a day.

13. Opioid receptor agonist according to claim 3, claim 6, claim 7 when depending on claim 6, or claim 10, wherein each of the daily dosages of the opioid receptor agonist is administered to the subject four times a day.

14. Opioid receptor agonist according to any of claims 11 to 13, wherein each of the daily dosages is administered to the subject in equally divided doses.

15. Opioid receptor agonist according to any of claims 1 to 14, wherein the opioid receptor agonist is buprenorphine or a derivative thereof, said opioid receptor agonist being preferably buprenorphine.
